# FASCICULE DE BREVET EUROPEEN

(11) **EP 2 346 875 B1**
(45) Date de publication et mention de la délivrance du brevet: **19.03.2014**
(21) Numéro de dépôt: 09741323.1
(22) Date de dépôt: 14.09.2009
(51) Int. Cl.: C07D 487/22, A61K 31/409, A61P 35/00

(54) **DÉRIVÉS DE METALLOPORPHYRINES, NANOPARTICULES LES COMPRENANT ET LEUR UTILISATION POUR LE TRAITEMENT PAR THÉRAPIE PHOTODYNAMIQUE**
METALLOPORPHYRINDERIVATE, NANOPARTIKEL DIE DIESE ENTHALTEN UND DEREN VERWENDUNG IN DER PHOTODYNAMISCHEN THERAPIE
METALLOPORPHYRIN DERIVATIVES, NANOPARTICLES COMPRISING THE SAME, AND USE THEREOF FOR PHOTODYNAMIC THERAPY

(30) Priorité: 15.09.2008 FR 0805034
(43) Date de publication de la demande: 27.07.2011
(73) Titulaire: Centre National de la Recherche Scientifique, 75016 Paris (FR); Institut Curie, 75248 Paris Cedex 05 (FR); Université Montpellier II, 34095 Montpellier Cedex 05 (FR); Université Mouloud Mammeri, 15000 Tizi Ouzou (DZ)
(72) Inventeur: BREVET, David, F-34090 Montpellier (FR); HOCINE, Ouahiba, Tizi Ouzou 15009 (DZ); DURAND, Jean-Olivier, F-34250 Palavas-Les-Flots (FR); MAILLARD, Philippe, F-78210 Saint-Cyr L'Ecole (FR); MORERE, Alain, F-34270 Les Matelles (FR); GARCIA, Marcel, F-34730 Prades-Le-Lez (FR); SMAÏHI, Monique, F-34170 Castelnau Le Lez (FR); GARY-BOBO, Magali, F-34090 Montpellier (FR)
(74) Mandataire: Majidi, Assieh
(86) Numéro de dépôt international: PCT/FR2009/001090
(87) Numéro de publication internationale: WO 2010/029232

(56) Documents cités:
- EP-A- 0 345 171
- WO-A-94/09003
- T.Y. OHULCHANSKYY ET AL.: "organically modified silica nanoparticles with covalently incorporated photosensitizer for photodynamic therapy of cancer" NANO LETTERS, vol. 7, no. 9, 2007, pages 2835-2842, XP002515000 cité dans la demande
- BONNET, MURIEL ET AL: "A new mesoporous hybrid material: porphyrin-doped aerogel as a catalyst for the epoxidation of olefins" ADVANCED FUNCTIONAL MATERIALS , 12(1), 39-42 CODEN: AFMDC6; ISSN: 1616-301X, 2002, XP001089357

## Description

L'invention a pour objet des nanovecteurs permettant simultanément de réaliser le ciblage, l'imagerie et le traitement par thérapie photodynamique de cellules cancéreuses. En particulier elle se fonde sur l'utilisation de nouvelles molécules dérivées de porphyrines, des nanoparticules siliciées les comprenant et leur utilisation en thérapie photodynamique.

La thérapie photodynamique se fonde sur l'utilisation de certaines molécules thérapeutiques appelées photosensibilisateurs, qui vont préférentiellement se localiser dans les tissus malins, et qui, lorsqu'elles sont activées à l'aide d'une source lumineuse de longueur d'onde appropriée, dans le domaine du visible ou du proche infra-rouge, transmettent leur énergie en excès à l'oxygène moléculaire les environnant. Cette activation entraîne la formation d'espèces oxygénées réactives telles que des radicaux libres et de l'oxygène singulet. Ces espèces oxygénées réactives, et plus particulièrement l'oxygène singulet, sont toxiques pour les cellules qui les entourent et entraînent la destruction des tissus malins dans leur proche environnement : elles oxydent les membranes cellulaires et entraînent ainsi des lésions irréversibles des cellules contenant le photosensibilisateur. La thérapie photodynamique se fonde sur une double sélectivité : en premier lieu l'irradiation sélective des tissus concernés et en second lieu la sélectivité relative du photosensibilisateur pour les tissus cibles. En l'absence d'irradiation, les photosensibilisateurs étant peu toxiques pour les cellules, leur diffusion dans l'organisme n'entraîne que peu d'inconvénients.

Un photosensibilisateur, pour pouvoir être employé *in vivo* doit posséder plusieurs qualités, et notamment, il doit pouvoir être facilement vectorisé vers les tissus cancéreux, il doit être hydrosoluble, facile à produire, non toxique en l'absence d'irradiation, stable vis-à-vis des enzymes circulantes, présentant un bon tropisme pour les cellules tumorales et il doit être éliminé rapidement des tissus sains. Toutefois, la plupart des molécules appartenant à la catégorie des photosensibilisateurs sont hydrophobes et leur introduction dans l'organisme, en particulier par voie parentérale, nécessite d'avoir recours à des formulations particulières, et notamment des formulations sous forme de suspensions colloïdales, de liposomes, de nanoparticules. Ces formulations permettent de stabiliser les photosensibilisateurs en milieu aqueux et de favoriser leur acheminement vers les tissus cibles, en particulier en utilisant des molécules de ciblage spécifiques. Une autre contrainte de ces formulations est de pouvoir conserver l'efficacité des photosensibilisateurs, c'est-à-dire leur capacité à transformer l'oxygène moléculaire qui les entoure en espèces oxygénées réactives. En effet, certaines formulations interagissent avec les états excités du photosensibilisateur et réduisent son efficacité.

La formulation des photosensibilisateurs dans des nanoparticules mésoporeuses à porosité contrôlée est apparue comme une voie prometteuse pour l'application en thérapie photodynamique : de telles formulations sont décrites notamment dans WO2004/067508 ; WO2008/030624 ; I. Roy et al., J. Am. Chem. Soc. 2003, 125, 7860-7865.

Toutefois, ces formulations entraînent bien souvent le relargage prématuré du photosensibilisateur dans l'organisme avant qu'il ne soit parvenu à sa cible.

Il est donc apparu nécessaire de trouver des moyens pour éviter ce relargage prématuré et une solution proposée a été d'élaborer des nanoparticules auxquelles le photosensibilisateur est lié de façon covalente.

Le document T.Y.Ohulchanskyy *et al.*, Nanolett. 2007, 2835 décrit des nanoformulations d'un photosensibilisateur pour la thérapie photodynamique, dans lesquelles le photosensibilisateur est lié de façon covalente à des nanoparticules de silice modifiées organiques (ORMOSIL). Le photosensibilisateur conserve ses propriétés spectroscopiques et fonctionnelles, les nanoparticules de petite taille, monodisperses, ont une bonne affinité pour les cellules cancéreuses et ont une efficacité cytotoxique élevée. Les photosensibilisateurs utilisés sont hydrophobes, ce qui contraint à faire la synthèse en milieu organique, avec des solvants dont l'industrie cherche aujourd'hui à éviter l'emploi à grande échelle.

Le document US2007/0218049 décrit des nanoparticules luminescentes auxquelles sont attachés des photosensibilisateurs de la famille des porphyrines. Les nanoparticules décrites dans ce document sont pleines et non poreuses, elles sont à base de molécules métalliques telles que CdS, CdSe, ZnO, et le photosensibilisateur est greffé en surface de ces nanoparticules par un lien cystéine.

Il subsistait donc le besoin de nouvelles nanoparticules à porosité contrôlée auxquelles soient liés de façon covalente des photosensibilisateurs, dans lesquelles ce dernier conserve ses propriétés spectroscopiques et fonctionnelles, ces nanoparticules pouvant être préparées par des méthodes simples et ne faisant pas appel à des composés toxiques et/ou polluants.

De telles nanoparticules ont pu être préparées grâce à la mise au point de nouveaux phosensibilisateurs répondant à la formule (I) ci-dessous.

L'invention a donc pour premier objet une molécule répondant à la formule (I) ci-dessous : 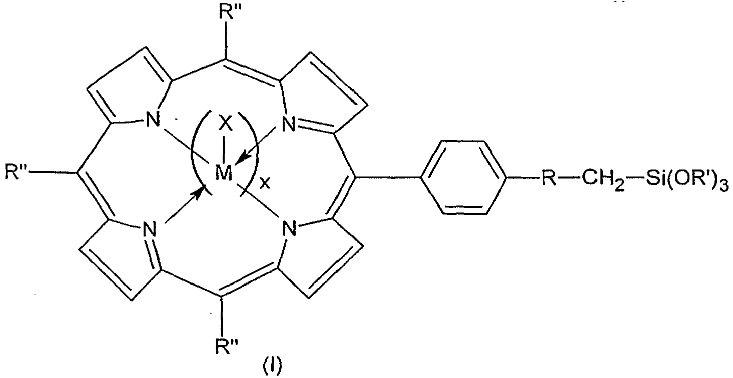 dans laquelle :
x représente un entier choisi parmi : 0 et 1,
M représente un atome de métal choisi parmi les métaux de transition,
X représente un groupement choisi parmi : un halogénure, un anion d'un acide carboxylique pharmaceutiquement acceptable,
R représente un groupement choisi parmi :
   une chaîne alkyle en C₁-C₁₅, éventuellement interrompue par un ou plusieurs groupements choisis parmi : un éther (-O-), une amine (-NH-), un thioéther (-S-), une cétone (-CO-), un ester (-CO-O-), un amide (-CO-NH-), une urée (-NH-CO-NH-), une thiourée (-NH-CS-NH-), un oxycarbonyl (-O-CO-O-), un carbamate (-NH-CO-O-), ou R représente 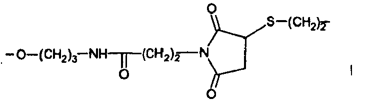
   R' représente un groupement choisi parmi : un alkyle en C₁-C₆, un phényle, un benzyle,
   R" représente un groupement choisi parmi : 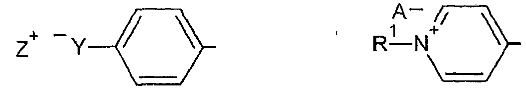
   et Z⁺ représente un cation organique ou minéral pharmaceutiquement acceptable,
   Y⁻ représente un groupement qui peut être choisi parmi : -COO⁻, -SO₃⁻,
   A⁻ représente un anion qui peut être choisi parmi : un halogénure, un anion d'un acide carboxylique pharmaceutiquement acceptable,
   R¹ représente un alkyle en C₁ à C₁₀.

Un groupement alkyle en C₁-C₁₀ est une chaîne hydrogénocarbonée, linéaire, ramifiée ou cyclique comportant de 1 à 10 atomes de carbone.

Lorsque x représente 0, le composé de formule (I) est un dérivé de porphyrine et les groupements (M-X) sont remplacés par deux atomes d'hydrogène.

Lorsque x représente 1, le composé de formule (I) est un dérivé de métalloporphyrine.

Dans la formule (I), les variables définies ci-dessus sont avantageusement choisies, de façon indépendante, suivant les règles suivantes :

De préférence, M représente un atome de métal choisi parmi : Zn, Pt, Pd, Mn, Gd, Ni, Cr, Ru.

De préférence, X représente un groupement choisi parmi : Cl⁻, Br⁻, I⁻, acétate, propionate, butyrate, ascorbate, benzoate, cinnamate, citrate, fumarate, glycolate, malonate, tartrate, malate, maléate, mandélate, tosylate, et encore plus préférentiellement : Cl⁻, Br⁻, I⁻, acétate, tosylate.

Avantageusement, R représente un groupement choisi parmi : une chaîne alkyle en C₁-C₁₀, éventuellement interrompue par un ou plusieurs groupements choisis parmi : un éther (-O-), une amine (-NH-), un thioéther (-S-), une cétone (-CO-), un ester (-CO-O-), un amide (-CO-NH-), une urée (-NH-CO-NH-), une thiourée (-NH-CS-NH-), un oxycarbonyl (-O-CO-O-), un carbamate (-NH-CO-O-),
et par exemple R peut être choisi parmi :

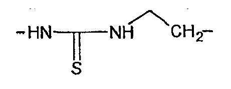

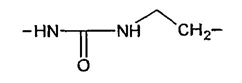

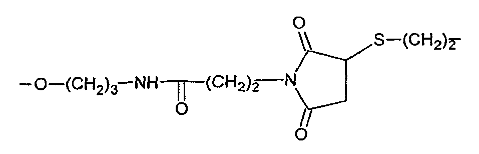

Avantageusement, R' représente un groupement choisi parmi : un alkyle en C₁-C₃, comme par exemple un méthyle, un éthyle, un n-propyle, un isopropyle, et de préférence un méthyle ou un éthyle.

Avantageusement R¹ représente un groupement choisi parmi : un alkyle en C₁-C₃, comme par exemple un méthyle, un éthyle, un n-propyle, un isopropyle, et encore plus avantageusement un méthyle.

De préférence, Z⁺ représente un cation qui peut être choisi parmi : K⁺, Li⁺, Na⁺, NH₄⁺.

De préférence A- représente un anion qui peut être choisi parmi : Cl⁻, Br⁻, I⁻, acétate, propionate, butyrate, ascorbate, benzoate, cinnamate, citrate, fumarate, glycolate, malonate, tartrate, malate, maléate, mandélate, tosylate, et encore plus préférentiellement. Cl⁻, Br⁻, I⁻, acétate, tosylate.

Les porphyrines (I) préférées appartiennent à la liste suivante :

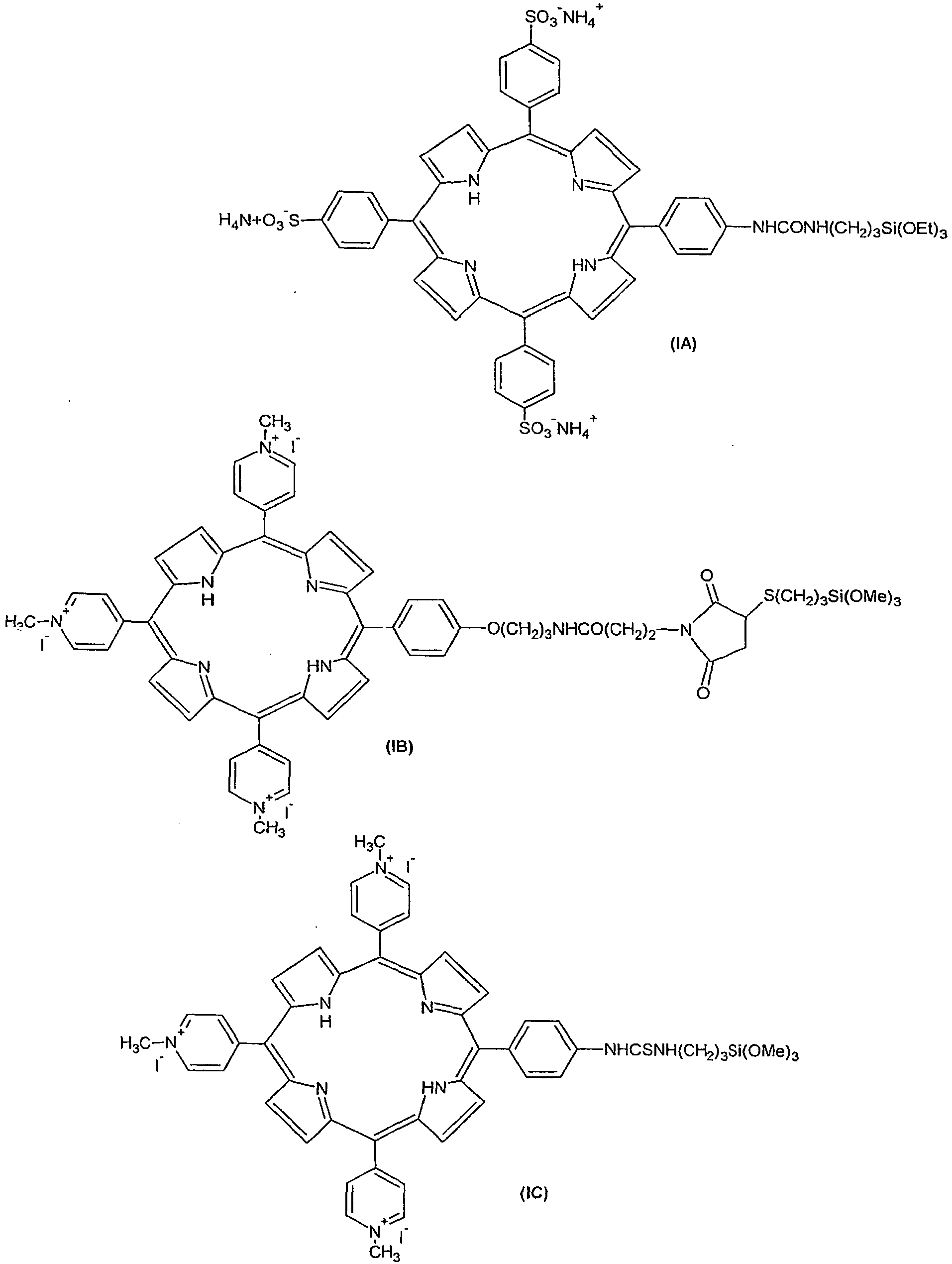

Les molécules répondant à la formule (I) sont des photosensibilisateurs hydrosolubles dotés d'une bonne capacité à transformer l'oxygène moléculaire qui les entoure en espèces oxygénées réactives. En raison de leur hydrosolubilité ils peuvent être facilement formulés dans des nanoparticules siliciées auxquelles ils sont liés de façon covalente. Et la synthèse de ces nanoparticules peut être réalisée en milieu essentiellement aqueux.

Lorsque R est choisi parmi : 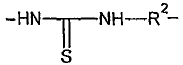 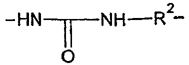 et R² représente un groupement choisi parmi une chaîne alkyle en C₁-C₁₅, éventuellement interrompue par un ou plusieurs groupements choisis parmi : un éther (-O-), une amine (-NH-), un thioéther (S-), une cétone (-CO-), un ester (-CO-O-), un amide (-CO-NH-), une urée (-NH-CO-NH-), une thiourée (-NH-CS-NH-), un oxycarbonyl (-O-CO-O-), un carbamate (-NH-CO-O-),
alors les molécules de formule (I) peuvent être préparées par un procédé tel que décrit dans le schéma 1 ci-dessous dans lequel on fait réagir une porphyrine porteuse d'une fonction amine (II) avec un composé isocyanatotrialcoxysilane (ou isothiocyanatoalcoxysilane lorsque R représente NH-CS-NHR²) :

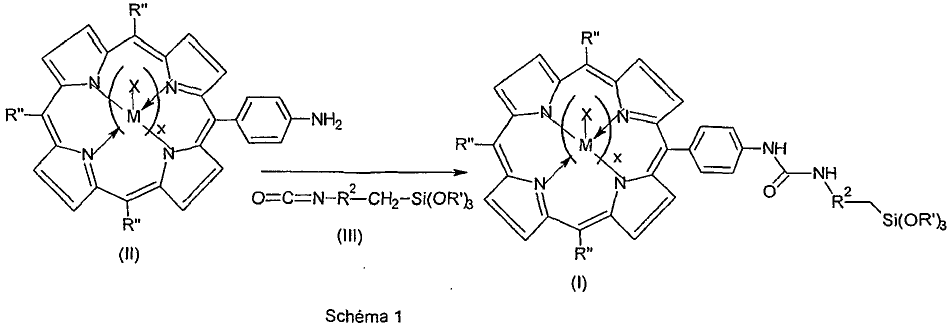

On peut également employer un procédé selon le schéma 2 dans lequel on fait réagir une porphyrine porteuse d'une fonction isocyanate (IV) (ou isothiocyanate lorsque R représente NH-CS-NHR²) avec un composé aminoalkyl trialcoxysilane (V) :

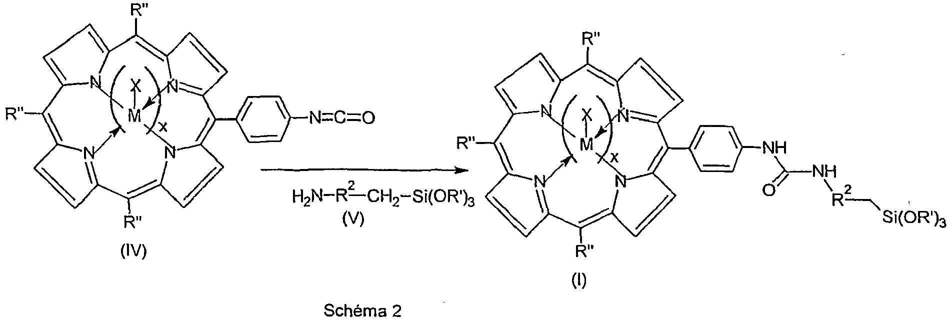

Lorsque R représente un groupement -CO-NH-R²- et R² représente un groupement choisi parmi une chaîne alkyle en C₁-C₁₅, éventuellement interrompue par un ou plusieurs groupements choisis parmi : un éther (-O-), une amine (-NH-), un thioéther (S-), une cétone (-CO-), un ester (-CO-O-), un amide (-CO-NH-), une urée (-NH-CO-NH-), une thiourée (-NH-CS-NH-), un oxycarbonyl (-O-CO-O-), un carbamate (-NH-CO-O-),

On peut alors réaliser un couplage à partir d'une porphyrine porteuse d'un groupement acide carboxylique suivant le schéma 3 ci-dessous :

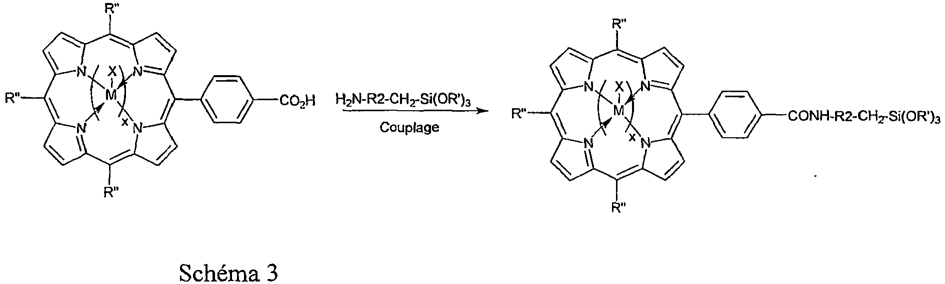

Lorsque R représente un groupement -NH-R²- ou -O-R²- et R² représente un groupement choisi parmi une chaîne alkyle en C₁-C₁₅, éventuellement interrompue par un ou plusieurs groupements choisis parmi : un éther (-O-), une amine (-NH-), un thioéther (S-), une cétone (-CO-), un ester (-CO-O-), un amide (-CO-NH-), une urée (-NH-CO-NH-), une thiourée (-NH-CS-NH-), un oxycarbonyl (-O-CO-O-), un carbamate (-NH-CO-O-),
on peut réaliser un couplage de type SN2, comme illustré respectivement sur le Schéma 4 et le Schéma 5.

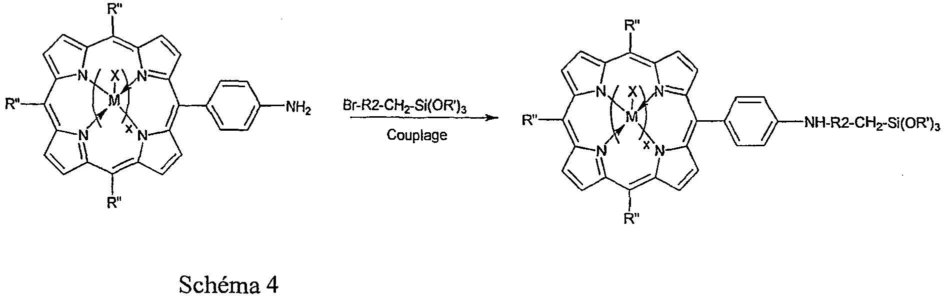

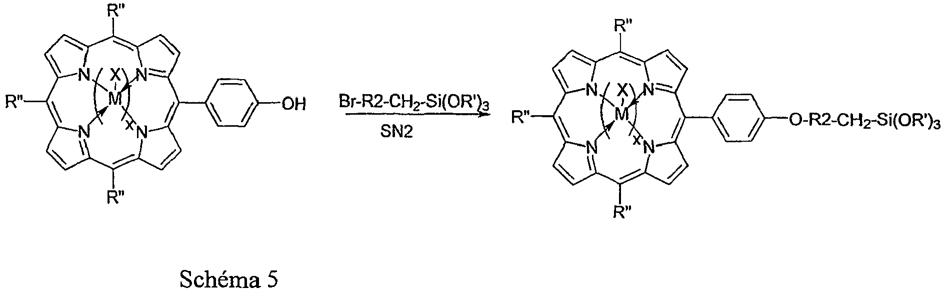

Lorsque R représente un groupement -O-CO-NH-R²- et R² représente un groupement choisi parmi une chaîne alkyle en C₁-C₁₅, éventuellement interrompue par un ou plusieurs groupements choisis parmi : un éther (-O-), une amine (-NH-), un thioéther (S-), une cétone (-CO-), un ester (-CO-O-), un amide (-CO-NH-), une urée (-NH-CO-NH-), une thiourée (-NH-CS-NH-), un oxycarbonyl (-O-CO-O-), un carbamate (-NH-CO-O-),
on peut former un lien carbamate en suivant la méthode illustrée sur le schéma 6 :

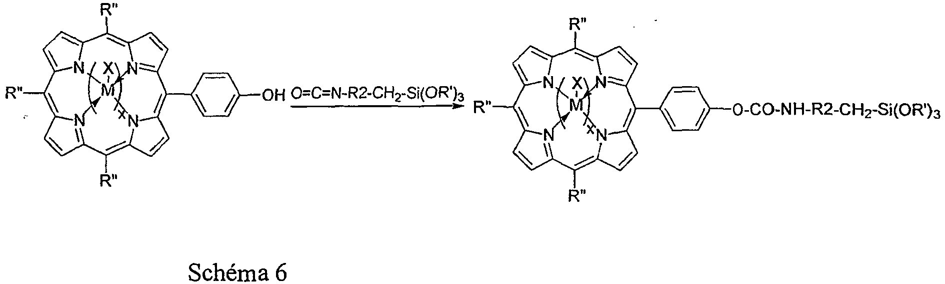

Pour la préparation des porphyrines de formule (I), les molécules de départ suivantes peuvent être employées :
Des porphyrines cationiques porteuses d'un groupement phényl-CO₂H sont décrites dans : H. Yamaguchi et al, Chem. Eur. J., 2004, 10, 6179. Des porphyrines anioniques porteuses d'un groupement -CO₂H sont décrites dans : US 4783529. Des porphyrines anioniques porteuses d'un groupement hydroxyphényl sont décrites dans : EP 891977.

La synthèse de porphyrines porteuses de groupements pyridine est décrite notamment dans EP-345171. De telles molécules peuvent être utilisées pour préparer des molécules de formule (I) comme illustré sur le schéma 7 ci-dessous :

On utilise comme produit de départ un composé de type porphyrine (VI) porteur de trois groupements pyridine et d'une fonction Q appropriée, précurseur d'un linker -R-CH₂-. Puis les groupements pyridine sont quaternisés pour donner (VII) et le groupement Q est transformé et greffé par un groupement trialcoxysilane pour donner (I).

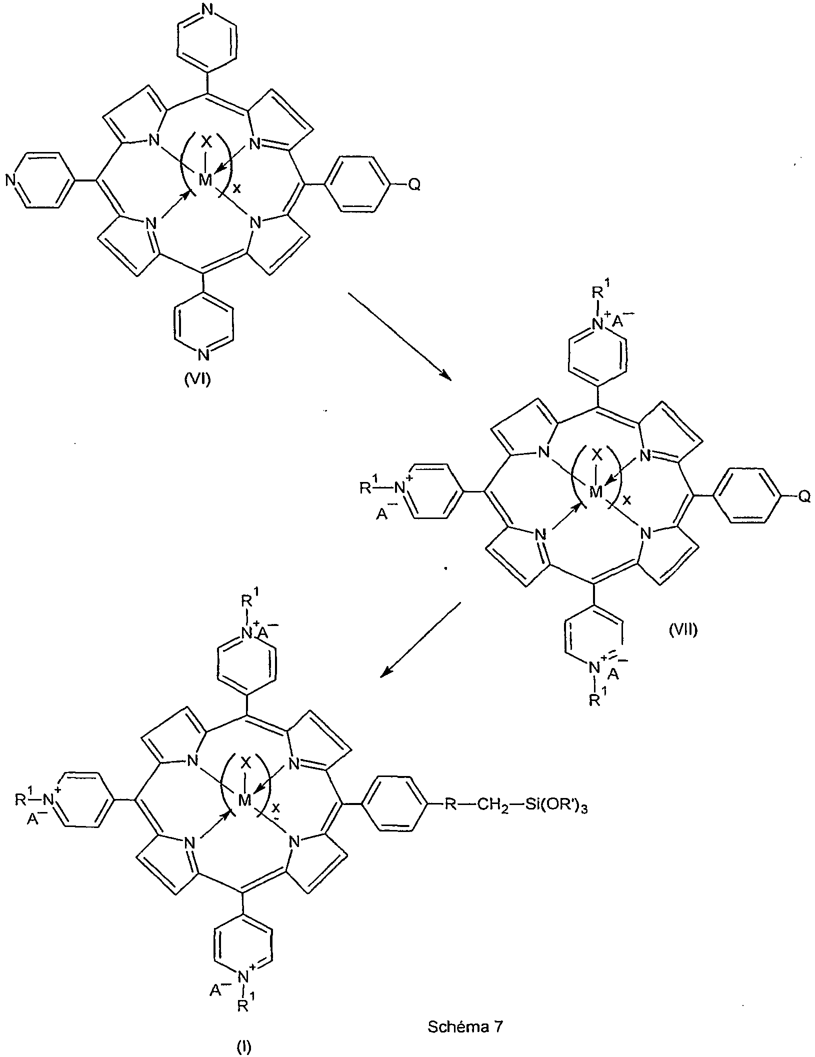

L'invention a encore pour objet les compositions de nanoparticules comportant au moins un photosensibilisateur répondant à la formule (I) ci-dessus. Ces nanoparticules sont avantageusement à porosité organisée.

Par nanoparticule à porosité organisée, ou à porosité structurée, on entend des nanoparticules dont les pores sont répartis de façon géométrique suivant un schéma régulier dans les trois dimensions de l'espace. Comme exemple de porosité organisée on peut citer une structure en nid d'abeille.

Le caractère structuré de la porosité peut être observé de différentes façons :
Les nanoparticules à porosité organisée ont un spectre de diffraction aux rayons X.

La méthode d'adsorption désorption d'azote (BET) permet également de caractériser une porosité structurée.

Le fait d'avoir une structure à porosité organisée facilite le transfert d'énergie des photosensibilisateurs vers l'oxygène et la libération des espèces réactives de l'oxygène, dont l'oxygène singulet, dans les cellules. En outre, les porphyrines (I) sont fluorescentes si les cations métalliques M sont diamagnétiques ou si x = 0, aussi les nanoparticules de l'invention permettent de procéder au marquage par fluorescence des zones où sont localisés les tissus cancéreux. Et certaines molécules de formule (I) comprennent un cation métallique paramagnétique (M dans la porphyrine) qui permet de suivre la répartition des nanoparticules dans l'organisme par RMN et IRM. Le fait que ces molécules de marquage soient dans une structure organisée, à porosité contrôlée, facilite l'analyse d'image.

De façon avantageuse, les nanoparticules de l'invention sont à base de silice. La fabrication de ces nanoparticules comporte une étape de polymérisation d'un précurseur silicié dans des conditions permettant le greffage covalent du photosensibilisateur (I) et la formation d'un réseau à porosité organisée.

Selon une première variante on choisit de fabriquer des nanoparticules de silice mésoporeuse, formées par polymérisation d'un précurseur silicié en présence de tensio actifs. Les nanoparticules de silice mésoporeuse ont l'avantage d'avoir une surface spécifique élevée, un volume et des pores de taille contrôlée. Les nanoparticules de l'invention sont avantageusement monodisperses. Les nanoparticules de silice mésoporeuse à porosité contrôlée de l'invention ont une taille de particules allant de 80 à 400 nm de diamètre, une surface spécifique allant de 800 à 1000 m²/g et des pores de taille allant de 2 à 6 nm. Ces nanoparticules sont avantageusement de type MCM41.

Des nanoparticules mésoporeuses à base de silice ainsi que leur procédé de synthèse ont été décrits notamment dans C.E.Fowler et al., Adv. Mater. 2001, 13, N°9, 649-652. Ce sont les mêmes modes opératoires qui sont employés dans la présente invention.

Le procédé de fabrication des nanoparticules de l'invention se caractérise par le fait que l'on fait polymériser le tétraéthoxysilane en solution aqueuse basique en présence d'un tensioactif tel que du bromure de cétyltriméthylammonium et en présence de la molécule de formule (I).

Selon une seconde variante on choisit de fabriquer des nanoparticules de silice microporeuses. Les nanoparticules microporeuses de l'invention sont de type silicalite et sont synthétisées en milieu basique en présence de la molécule de formule (I), du tétraéthoxysilane et d'un agent structurant de type ammonium quaternaire (comme par exemple de l'hydroxyde de tétrapropylammonium). Les nanoparticules de l'invention sont avantageusement monodisperses. Les nanoparticules de l'invention sont alors microporeuses avec un diamètre compris entre 30 et 80 nm, une surface spécifique allant de 100 à 450 m²/g et des diamètres de pores allant de 2 à 20 Å.

Des nanoparticules microporeuses à base de silice ainsi que leur procédé de synthèse ont été décrites notamment dans T. Doussineau et al., Eur. J. Inorg. Chem. 2006, 2766-2772. Ce sont les mêmes modes opératoires qui sont employés dans la présente invention.

Les procédés de production de ces nanoparticules conduisent à la formation d'une liaison covalente entre les molécules de formule (I) et les nanoparticules, ce qui évite le relargage des molécules de formule (I) dans l'organisme et favorise leur acheminement de façon spécifique vers leur cible biologique.

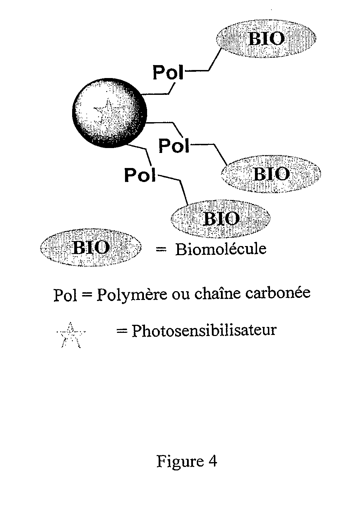

De préférence les nanoparticules de l'invention sont greffées sur leur surface par des molécules de ciblage spécifiques des tissus néoplasiques, c'est-à-dire des biomolécules dont les récepteurs sont surexprimés par les cellules cancéreuses, ou à la surface des cellules cancéreuses. Ces molécules de ciblage facilitent le transfert des nanoparticules vers leur cible biologique. D'une façon générale, ces molécules de ciblage peuvent être choisies parmi : l'acide folique, les peptides, les hydrates de carbone. Elles peuvent être greffées sur les nanoparticules de l'invention par l'intermédiaire d'un linker polymérique comme illustré sur la figure 4.

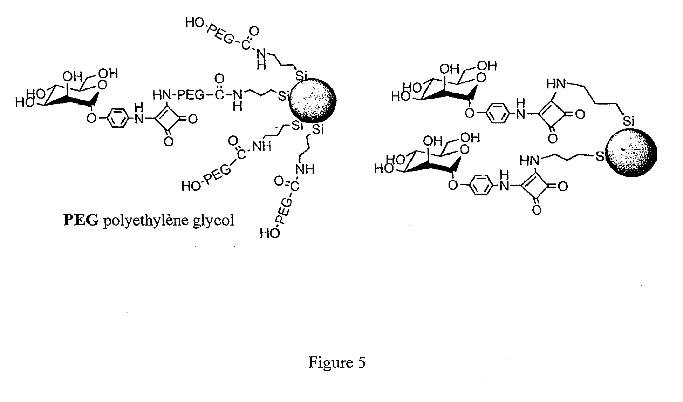

Parmi les molécules de ciblage qui peuvent être greffées sur les nanoparticules de l'invention on peut mentionner :
- des dérivés de sucre comme le mannose, qui peut être greffé sur des nanoparticules siliciées à l'aide d'un linker polyéthylèneimine ou polyéthylène glycol PEG. Le greffage du mannose sur des nanoparticules de silice mésoporeuse est décrit notamment dans I. Young Park et al., International Journal of Pharmaceutics, 359, 2008, 280-287, et l'on peut employer les mêmes modes opératoires. Une autre voie consiste à utiliser le phénylsquarate-α-mannose comme illustré sur la figure 5.
- des peptides tels que des peptides à cible hormonale (LH-RH).
- des glycodendrimères portant plusieurs dérivés monosaccharides ou disaccharides tels que mannose, mannose-6-phosphate, glucose, galactose,...

Des méthodes de greffage des nanoparticules microporeuses à base de silice par des biomolécules ont été décrites notamment dans T. Doussineau et al., Eur. J. Inorg. Chem. 2006, 2766-2772 et l'on peut employer les mêmes modes opératoires.

L'invention a encore pour objet un procédé de fabrication d'un médicament destiné au traitement et/ou à la prévention et/ou à la détection des tumeurs comprenant l'étape de fabrication des nanoparticules décrites ci-dessus.

La production d'oxygène singulet et donc la destruction des cellules cancéreuses est induite par excitation photonique : Après irradiation des nanoparticules par une source lumineuse, l'oxygène singulet généré permet de détruire les cellules tumorales. L'irradiation est faite à une longueur d'onde comprise entre 630 nm et 680 nm à une puissance allant de 2 à 10 mW/cm². Les résultats obtenus montrent que les nanoparticules synthétisées sont efficaces pour générer de l'oxygène singulet en solution avec un rendement quantique compris entre 30 et 90%. Ces nanoparticules doivent permettre une vectorisation des actifs et leur endocytose dans les cellules tumorales après fonctionnalisation en surface par des biomolécules.

Parmi les différents cancers que l'on peut envisager de traiter avec les nanoparticules de l'invention, on peut citer : le rétinoblastome (lignées cellulaires Y-79), le cancer du colon (lignées cellulaires HT29), de l'épiderme (A 431), du poumon (A 549), du sein (MDA-MB-231, MCF-7), du col de l'utérus (HeLa) de l'ovaire (PEO14) ainsi que l'ensemble des tumeurs solides qui inclut, mais n'est pas limité aux cancers du cou et de la tête, aux cancers digestifs et des organes sexuels, et toute tumeur bénigne ou cancéreuse qui peut être illuminée.

Après endocytose les lignées cellulaires sont irradiées dans une des bandes d'absorption du photosensibilisateur, et l'efficacité des nanoparticules est évaluée par test MTT.

Les compositions de nanoparticules de l'invention peuvent être administrées localement ou par voie systémique. L'administration locale peut être effectuée notamment par injection de la composition de nanoparticule à proximité de la zone tumorale. Dans le cas des tumeurs superficielles, les compositions de nanoparticules peuvent être administrées par voie topique, sous une forme galénique appropriée (solution, suspension, pâte, patch). L'administration par voie générale peut être mise en oeuvre par voie intraveineuse, intramusculaire, sous-cutanée, intrapéritonéale ou rectale. De telles formulations et leur mode de mise en oeuvre sont bien connus de l'homme du métier.

Le dosage de la composition en actif de formule (I) est adapté en fonction du poids et de l'âge du patient, de la nature, de la localisation et du stade de développement de la tumeur, de la voie d'administration choisie et de la dose d'irradiation utilisée.

La composition peut comprendre tout autre actif connu pour le traitement des tumeurs et/ou de leurs symptômes. Elle comprend les composants classiques de la galénique adaptés au mode d'administration choisi.

L'invention a encore pour objet les nanoparticules décrites ci-dessus, pour leur utilisation comme médicament, en particulier pour la prévention et/ou le traitement de tumeurs et cancers.

### PARTIE EXPERIMENTALE

### Figures :

Figure 1 : Diffractogramme aux rayons X des nanoparticules de silicalite encapsulant une porphyrine anionique.
Figure 2 : Cliché TEM d'une nanoparticule mésoporeuse à porosité organisée.
Figure 3 : Spectre UV des nanoparticules DB015 (NP-PS), DB016 (NP-Mannose) et de l'arylsquarate-α-mannose (Sq-Mannose).
Figure 4 : nanoparticules de l'invention greffées par des molécules de ciblage
Figure 5 : exemple de nanoparticules fonctionalisées par le mannose
Figures 6 à 14: Résultats biologiques sur l'efficacité cytotoxique de différentes nanoparticules encapsulant des photosensibilisateurs sur des lignées cellulaires de cancer du sein (MDA-MB-231), de l'ovaire (POE14), de la prostate (LNCaP) et de rétinoblastome (Y-79) après excitation monophotonique.

### I- Synthèse des molécules de formule (I) :

### A- Synthèse de la porphyrine cationiques hydrosoluble 8

### - Schéma de synthèse :

Le composé **3** (1 équivalent) obtenu par la méthode décrite par Diane L. Dick et al, J. Am. Chem. Soc. 1992, 114, 2664-2669, est condensé selon la procédure standard de Adler, avec le pyridinaldéhyde (3 équivalents) et le pyrrole (4 équivalents) pour donner, après chromatographie, la porphyrine **4** avec un rendement de 5,7 %, comme décrit par Martine Perrée-Fauvet et al pour le composé analogue *méta* Tetrahedron. 1996, 52, 13569-13588. Le dérivé **5** est obtenu quantitativement à partir du composé **4** par traitement avec de l'hydrazine dans l'eau à reflux. La réaction de la porphyrine **5** et de l'acide **6** peptidique dans le dichlorométhane, en présence de 1-hydroxybenzotriazole hydrate (HOBt), 1-(3-diméthylaminopropyl)-3-éthylcarbodiimide chlorhydrate (EDC) et de triéthylamine comme agents de couplage, conduit au composé **7** (rend : 40 %) (Daniel H. Rich, et al., J. Med. Chem. 1975, 18, 1004-1010). Ce dernier composé est traité par un large excès d'iodure de méthyle pour donner quantitativement la porphyrine hydrosoluble 8.

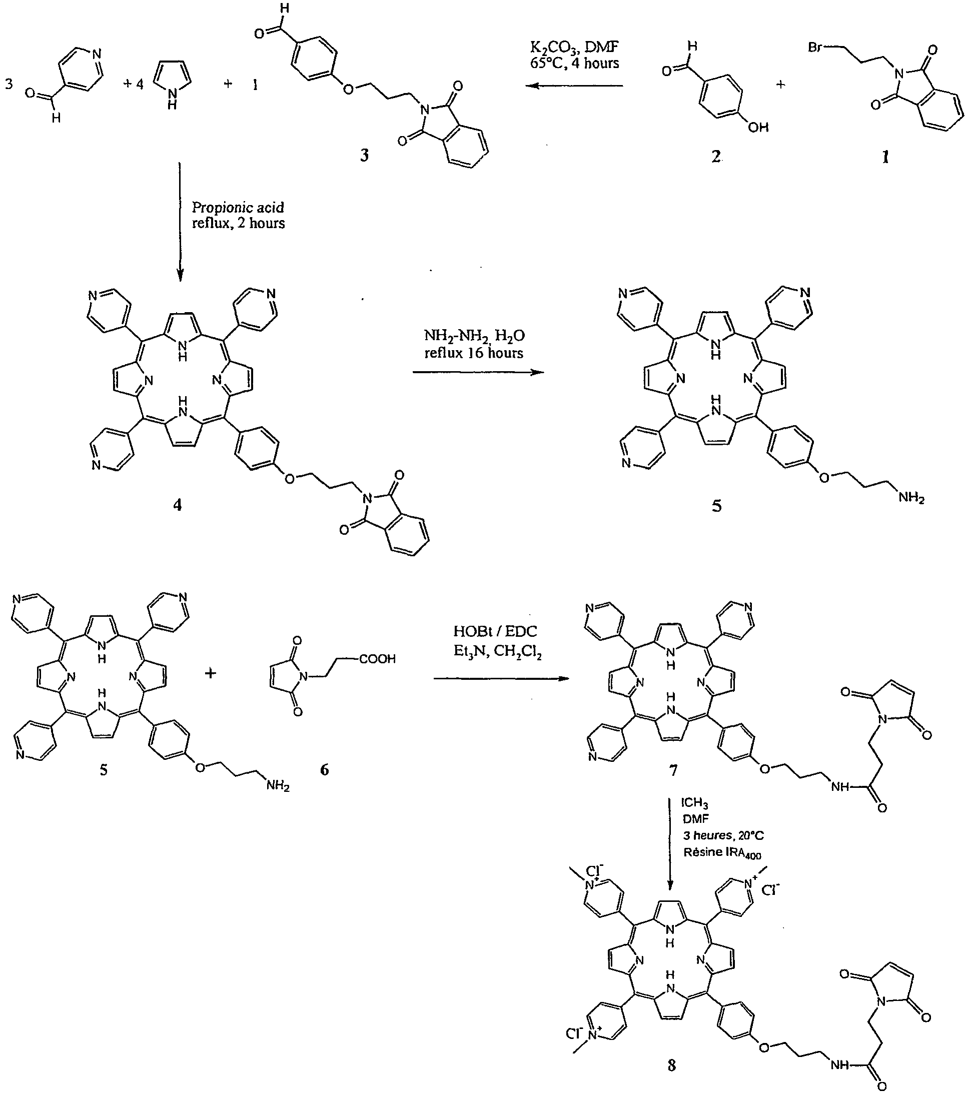

### - Modes opératoires:

### 5-[p-(3-isoindoline-1',3'-dione-propoxy)-phényle]-10,15,20-tri-p-pyridyl-porphyrine 4

La porphyrine **4** est préparée à partir de l'aldéhyde 3 (6,2 g, 20 mmol) et la 4-pyridinecarboxaldéhyde (6,4 g, 60 mmol) qui sont dissous dans l'acide propionique bouillant (400 mL). Puis le pyrrole (5,36 g, 80 mmol) est ajouté goutte-à-goutte. Le reflux est maintenu 2 h. La solution est évaporée sous vide. Une première purification par chromatographie sur gel de silice élué par un mélange CH₂Cl₂/EtOH (100/5, v/v) est effectuée afin d'éliminer un maximum d'impuretés. Une seconde, éluée avec du CH₂Cl₂ puis une quantité croissante d'éthanol (0 à 10%) permet la séparation des six porphyrines. La 5,10,15-tripyridyl-20-phénylporphyrine **4** est éluée (troisième fraction) avec un mélange CH₂Cl₂/EtOH (94/6, v/v) et est obtenue après cristallisation, par un mélange dichlorométhane/méthanol, sous forme de cristaux bleus (rendement : 5,7%). Spectre UV-vis dans le CH₂Cl₂ : λₘₐₓ, nm (DO) : 418,5 (1), 514,5 (0,61), 549 (0,31), 589 (0,25), 646 (0,16). RMN ¹H (300 MHz, CDCl₃) δ 9,05 (m, 6 H, *méta-*pyridine), 8,95 (d, J = 4,7 Hz, 2 H, pyrrole), 8,85 (s, 4 H, pyrrole), 8,81 (d, J = 5 Hz, 2 H, pyrrole), 8,16 (d, J = 5,8 Hz, 6 H, *ortho*-pyridine), 8,07 (d, J = 8,4 Hz, 2 H, *méta-*phényle), 7,93 (dd, J = 3,1, 5,4 Hz, 2 H, phthalimide), 7,76 (dd, J = 3,0, 5,4 Hz, 2 H, phthalimide), 7,12 (d, J = 8 Hz, 2 H, *ortho*-phényle), 4.35 (t, 8 Hz, 2 H, O-CH₂), 4.09 (t, 8 Hz, 2 H, N-CH₂), 2.40 (m, 2 H, CH₂), -2.87 (s, 2 H, NH).

### 5-[p-(3-aminopropoxy)-phényle]-10,15,20-tri-p-pyridylporphyrine 5

Un mélange de porphyrine **4** (0,930 g, 1,13 mmol) et de monohydrate d'hydrazine (0,71 mL, 23 mmol) est porté à reflux pendant 16 h, puis agité, à température ambiante, 24 h. La phthalhydrazide est précipitée par addition de HCl (solution 10%) et filtrée. La solution est neutralisée par addition de NaOH (solution 10%). La porphyrine est extraite de la phase aqueuse avec un mélange CH₂Cl₂/EtOH (95/5, v/v). Après séchage sur sulfate de sodium, filtration et évaporation à sec, la porphyrine **5** (0,760 g) est obtenue pure sous la forme d'une poudre bleue (rendement : 97 %). Spectre UV-vis dans le CH₂Cl₂ : λₘₐₓ, nm (ε L.mmol⁻¹.cm⁻¹) : 418 (224,9), 515 (13,1), 550 (6,5), 590 (5,4), 647 (4,1). RMN ¹H (300 MHz, CDCl₃) δ 9,05 (m, 6 H, *méta*-pyridine), 8,95 (d, J = 4,7 Hz, 2 H, pyrrole), 8,85 (s, 4 H, pyrrole), 8,81 (d, J = 5 Hz, 2 H, pyrrole), 8,16 (d, J = 5,8 Hz, 6 H, *ortho*-pyridine), 8,10 (d, J = 8,4 Hz, 2 H, *méta*-phényle), 7,3 (d, J = 8 Hz, 2 H, *ortho*-phényle), 4,38 (t, J = 8 Hz, 2 H, O-CH₂), 3,09 (t, J = 8 Hz, 2 H, N-CH₂), 2,14 (m, 2 H, CH₂), -2.87 (s, 2 H, NH).

### 5-{p-[3-(2',5'-dioxo-2',5'-dihydro-1H-pyrrol-1'-yl)-N-3-phénoxypropyl)propanamide]-phényle}-10,15,20-tri-p-pyridyl-porphyrine 7

Le composé 5 (160 mg, 0,23 mmol) est dissout dans CH₂Cl₂ (40 mL). Le HOBt (48 mg, 0,35 mmol), le EDC (67 mg 0,35 mmol), la triéthylamine (48 mL, 0,35 mmol) et l'acide 5-(2,5-dioxo-2,5-dihydro-pyrrol-1-yl)-propionique (80mg, 0,46 mmoles) sont ajoutés. Le mélange est agité sous argon, pendant trois heures, à température ambiante. La fin de la réaction est contrôlée par chromatographie analytique sur couche mince de gel de silice éluée par un mélange CH₂Cl₂/éthanol (90/10, v/v). La solution est diluée par un mélange de CH₂Cl₂-éthanol (95/5, v-v) et lavée avec de l'eau (x 3), séchée sur sulfate de sodium, filtrée puis concentrée sous vide. Le produit est purifié par chromatographie sur couche mince (Al₂O₃) élué avec un mélange de CH₂Cl₂/méthanol (9/1, v/v). Le composé 7 est obtenu sous la forme d'une poudre bleue après cristallisation dans un mélange CH₂Cl₂/éthanol/heptane (110 mg, rendement : 57 %). Spectre UV-vis dans le CH₂Cl₂ : λₘₐₓ, nm (ε L.mmol⁻¹.cm⁻¹) : 418 (190,6), 516 (13,7), 550 (8,3), 591 (7,1), 652 (6,7). Spectre de masse Electro spray : cal. pour C₅₁H₃₉N₉O4 841.9 trouvé 842,66 M + 1. RMN ¹H (300 MHz, CDCl₃) δ 9,04 (d, J = 5,6 Hz, 6 H, *méta*-pyridine), 8,92 (d, J = 5 Hz, 2 H, pyrrole), 8,83 (s, 4 H, pyrrole), 8,80 (d, J = 4,7 Hz, 2 H, pyrrole), 8,13 (d, J = 5,7 Hz, 6 H, *ortho*-pyridine), 8,10 (d, J = 8,5 Hz, 2 H, *méta*-phényle), 7,30 (d, J = 8,6 Hz, 2 H, *ortho*-phényle), 4,32 (t, 8 Hz, 2 H, O-CH₂), 3,92 (t, J = 7,4 Hz, 2 H, CH₂-N), 3,63 (t, J = 7,8 Hz, 2 H, CH₂-NH-CO), 2,62 (t, J = 7 Hz, 2 H, CH₂-CO-NH), 2,19 (t, 2 H, CH₂), -2,87 (s, 2 H, NH). ¹³C NMR (300 MHz, CDCl₃) δ 170,6 (CON), 169,7 (CO-NH), 158,8 (*para*-phényle), 150 (pyridine), 148,4 (*méta*-pyridine), 135,7 (*ortho*-phényle), 134,3 (éthylène), 134 (phényle), 131 (pyrrole), 129,4 (*ortho*-pyridine), 121,6 (*méso*-C), 117,4 (*méso*-C), 116,7 (*méso*-C), 112,8 (*méta-*phényle), 66,6 (C-O-), 37,5 (C-NHCO), 34,9 (C-N), 34,3 (C-CONH), 29,2 (C-C-O).

### 5-{p-[3-(2',5'-dioxo-2',5'-dihydro-1H-pyrrol-1'-yl)-N-3-phénoxypropyl)propanamide]-phényle}-10,15,20-tri-p-pyridynium-porphyrin tri chlorure 8

La porphyrine 7 (68 mg, 0,08 mmol) et l'iodure de méthyle (1 mL) sont dissous dans le diméthyl formamide (20 mL) et agités à température ambiante pendant trois heures. La solution est concentrée sous vide puis diluée par du méthanol (10 mL). La résine IRA 400 (0.9 g) est ajoutée à la solution puis la suspension est agitée doucement pendant 1 heure 30. La solution est filtrée puis évaporée. Le composé pur est obtenu sous forme d'une poudre bleue après cristallisation dans un mélange méthanol/diéthyl éther (80 mg, rendement : 100 %). Spectre UV-vis dans le MeOH : λₘₐₓ, nm (µL.mmol⁻¹.cm⁻¹) : 427 (98,3), 518 (12), 557 (6,1), 593 (4,1), 652 (2,5). Spectre de masse MALDI-TOF : calc pour C₅₄H₄₈N₉O₄Cl₃ 993,38 trouvé 886,44, M - 3Cl⁻, 887,44, M + H - 3Cl⁻.

### B- Synthèse de la porphyrine anionique hydrosoluble 9

Ce composé est préparé par la méthode décrite par Kruper et *al.*, J. Org. Chem. 1989, *54*, 2753-2756 à partir de la 5,10,15,20-tétraphényl porphyrine avec un rendement global de 43 %.

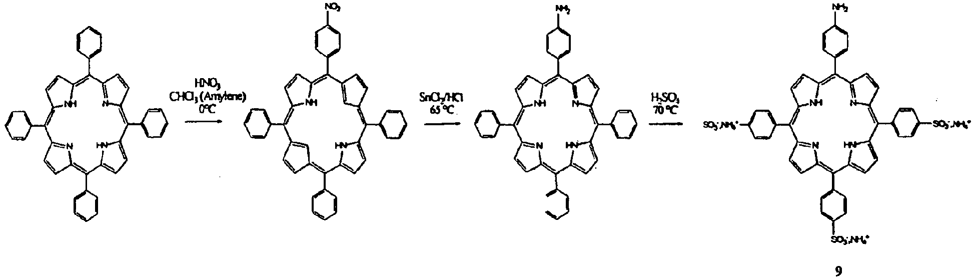

### II- Synthèse des nanoparticules :

### A- Synthèse de nanoparticules mésoporeuses encapsulant une porphyrine anionique - Nanoparticules DB 003, DB 005 :

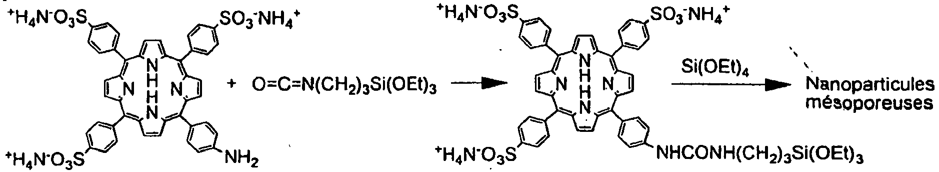

DB 003 : 5 mg de la porphyrine **9** (5,44.10⁻³ mmol) sont dissous dans 1 mL d'EtOH par passage pendant 15 min aux ultrasons. 5 équivalents (2,72 10⁻² mmol) d'isocyanatopropyltriéthoxysilane sont ajoutés ainsi que 4 équivalents (2,17 10⁻² mmol) de diisopropyléthylamine. La solution est maintenue à température ambiante pendant 12 heures.

686 mg (1,8 10⁻³ mol) de bromure de cétyltriméthylammonium (CTAB) sont dissous dans 40 mL de soude 0,2M à 25°C. La solution précédemment préparée est ajoutée puis le tétraéthoxysilane (3,5 mL, 1.57 10⁻² mmol) est ajouté goutte à goutte. Après 40 secondes, 260 mL d'eau déionisée sont ajoutés. La solution est maintenue 6 minutes sous agitation puis rapidement neutralisée à pH 7 par l'addition de HCl 0,2 M (environ 50 mL). Les nanoparticules sont récupérées par centrifugation minutes 20000 tours-minute), remises en suspension dans EtOH aux ultrasons, centrifugées. Le tensioactif est extrait par traitement avec 30 mL de solution EtOH/HCl 12N (4/1) durant 2h à 60°C. Après centrifugation, l'opération est répétée deux fois, puis les nanoparticules remises en suspension dans l'eau et centrifugées jusqu'à obtenir un pH neutre (5 fois).

Alternativement le tensioactif peut être éliminé par traitement au nitrate d'ammonium (Chem Mater, 2004, 10, 1961) : 300 mg de NH₄NO₃ sont dissous dans 150 mL d'EtOH 95%. 500 mg de nanoparticules sont mis en suspension dans cette solution et traités aux ultrasons pendant 15 minutes, puis la solution est placée à 60°C pendant 15 min. La suspension est récupérée par centrifugation, redispersée aux ultrasons dans EtOH, centrifugée. Le protocole d'extraction du tensioactif est répété une fois.

La microscopie électronique à transmission (TEM) montre la présence d'un réseau hexagonal de mésopores avec un diamètre des nanoparticules de l'ordre de 100 nm.

La diffusion quasi-élastique de la lumière (DLS) confirme le diamètre hydrodynamique de 150 nm.

La BET indique une surface spécifique de 862 m²/g et un diamètre de pores de 2 nm.

L'analyse par spectroscopie d'absorption UV-visible dite analyse UV (EtOH) confirme la présence de la porphyrine encapsulée de manière covalente, on obtient une charge de 3,3 µmol de porphyrine par g de nanoparticules.

La capacité des nanoparticules à générer de l'oxygène singulet est évaluée par phosphorescence de celui-ci après irradiation dans une des bandes d'absorption du photosensibilisateur et en particulier à 421 nm de 3 mg de nanoparticules dans 5 mL d'EtOH absolu. Le rendement quantique de formation de l'oxygène singulet est de 92%.

Le même mode opératoire est utilisé pour synthétiser les nanoparticules DB 005 mais on utilise 10 mg (10,88 10⁻³ mmol) de la porphyrine 9, 13,4 µl d'isocyanatopropyltriethoxysilane, 7,4 µl de diisopropyléthylamine.

La microscopie électronique à transmission (TEM) montre la présence d'un réseau hexagonal de mésopores avec un diamètre des nanoparticules de l'ordre de 100 nm.

La diffusion quasi-élastique de la lumière (DLS) confirme le diamètre hydrodynamique de 200 nm.

La BET indique une surface spécifique de 891 m²/g et un diamètre de pores de 2 nm.

L'analyse par spectroscopie d'absorption UV-visible dite analyse UV (EtOH) confirme la présence de la porphyrine encapsulée de manière covalente, on obtient une charge de 6,94 µmol de porphyrine par g de nanoparticules.

La capacité des nanoparticules à générer de l'oxygène singulet est évaluée par phosphorescence de celui-ci après irradiation dans une des bandes d'absorption du photosensibilisateur et en particulier à 421 nm de 3 mg de nanoparticules dans 5 mL d'EtOH absolu. Le rendement quantique de formation de l'oxygène singulet est de 32%.

### B- Greffage de l'aminopropyltriéthoxysilane (APTS) en surface des nanoparticules - Nanoparticules DB 015, DB 019:

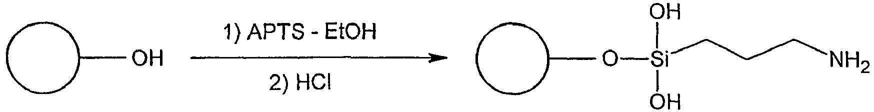

DB 015 : 250 mg des nanoparticules DB 003 sont mises en suspension dans 6 mL d'H₂O déionisée aux ultrasons pendant 30 min. Puis une solution de 2,5 mL d'EtOH et 391 µl d'APTS est ajoutée au goutte à goutte. On ajoute HCl 0,2M jusqu'à pH = 6. La réaction est agitée pendant 20h, centrifugée pendant 15 min. à 20000 tours. Les nanoparticules sont lavées par EtOH (ultrasons + centrifugation) puis lavées par EtOH au Soxhlet durant 20h. On obtient m = 206 mg de nanoparticules.

Les fonctions amines sont caractérisées par un test qualitatif à la ninhydrine.

L'analyse UV (EtOH) indique que la porphyrine est toujours présente et n'a pas été altérée durant la réaction.

La BET indique que la surface spécifique a diminué à 481 m²/g, du fait de la présence des groupements aminopropyle à la surface des nanoparticules mais aussi dans les pores.

Le dosage de l'APTS est effectué par microanalyse et RMN ²⁹Si du solide. On obtient une charge aminée de 2,2 mmol/g.

Le même mode opératoire est utilisé pour synthétiser les nanoparticules DB 019 à partir des nanoparticules DB 005,

La BET indique que la surface spécifique a diminué à 545 m²/g, du fait de la présence des groupements aminopropyle à la surface des nanoparticules mais aussi dans les pores.

### C- Synthèse des nanoparticules de silicalite encapsulant une porphyrine anionique - Nanoparticules DB 008 :

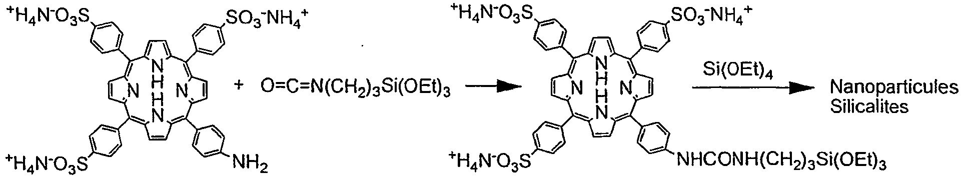

4 mg de la porphyrine **9** (4,35.10⁻³ mmol) sont dissous dans 1 mL d'EtOH par passage pendant 15 min aux ultrasons. 5 équivalents (4,5 µL) d'isocyanatopropyltriéthoxysilane sont ajoutés ainsi que 4 équivalents (3,04 µL) de diisopropyléthylamine. La solution est maintenue à température ambiante pendant 12 h, puis 14 mL d'hydroxyde de tétrapropylammonium 1M dans l'eau, 8,4 mL de TEOS et 2 mL d'eau sont ajoutés et la réaction est maintenue à température ambiante durant 24 h. La solution est ensuite mise à l'étuve à 80°C sans agitation pendant 48 h. Après refroidissement la solution est centrifugée 3 fois 20 minutes à 20000 tours-minute. Les nanoparticules sont ensuite redispersées dans l'eau et centrifugées. L'extraction de l'agent structurant est effectuée par une solution EtOH/HCl 12N (4/1) durant 2h à 60°C. Deux cycles sont effectués. Les nanoparticules sont ensuite lavées par H₂O 6 fois (6 cycles dispersion dans l'eau aux ultrasons centrifugation) jusqu'à obtenir un pH de 3,5. Les nanoparticules sont ensuite lavées deux fois par EtOH.

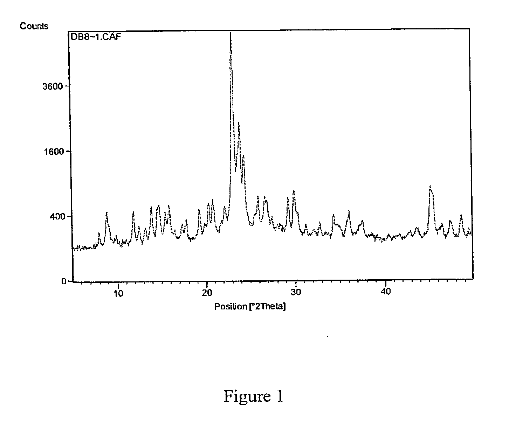

Les nanoparticules sont analysées par rayons X sur poudres et l'analyse montre la présence d'un réseau structuré caractéristique des zéolithes (figure 1).

La DLS donne un rayon hydrodynamique de 68 nm.

La BET donne une surface spécifique de 300 m²/g.

La capacité des nanoparticules à générer de l'oxygène singulet est évaluée par phosphorescence de celui-ci après irradiation dans l'une des bandes d'absorption du photosensibilisateur et en particulier à 421 nm de 3 mg de nanoparticules dans 5 mL d'EtOH absolu. Le rendement quantique de formation de l'oxygène singulet est de 31%.

### D- Synthèse des nanoparticules de silicalite encapsulant une porphyrine cationique - Nanoparticules DB 011 :

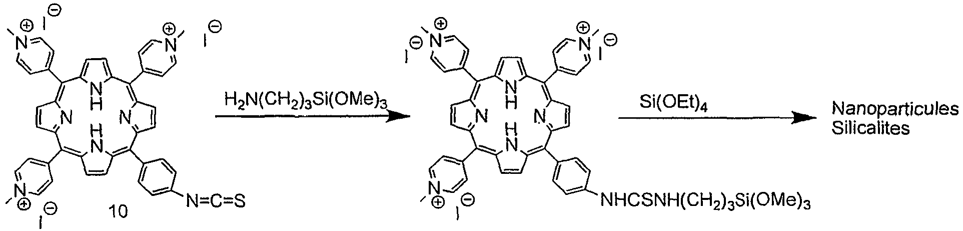

4,9 mg de la porphyrine 10 sont dissous dans 1 mL de EtOH absolu aux ultrasons. 2 µL d'aminopropyltriéthoxysilane sont ajoutés et la réaction est maintenue à température ambiante pendant une nuit. Dans un flacon en polyéthylène on dissout sous forte agitation la réaction précédente avec 14 mL d'hydroxyde de tétrapropylammonium 1M dans l'eau, 8,4 mL de tétraéthoxysilane et 2 mL d'H₂O. La solution est agitée pendant 24h à température ambiante puis est mise à l'étuve à 80°C sans agitation. Après deux jours la réaction est refroidie puis centrifugée. Les nanoparticules sont redispersées dans l'eau aux ultrasons puis centrifugées (3 cycles). L'extraction de l'agent structurant est effectuée par une solution EtOH/HCl 12N (4/1) durant 2h à 60°C. Deux cycles sont effectués. Les nanoparticules sont ensuite lavées par H₂O 6 fois (6 cycles : dispersion dans l'eau aux ultrasons - centrifugation) jusqu'à obtenir un pH de 3,5. Les nanoparticules sont ensuite lavées deux fois par EtOH.
Diffraction rayons X : réseau structuré
DLS : 92 nm
BET : 100 m²/g

### E- Synthèse de nanoparticules mésoporeuses encapsulant une porphyrine cationique - Nanoparticules OH21 :

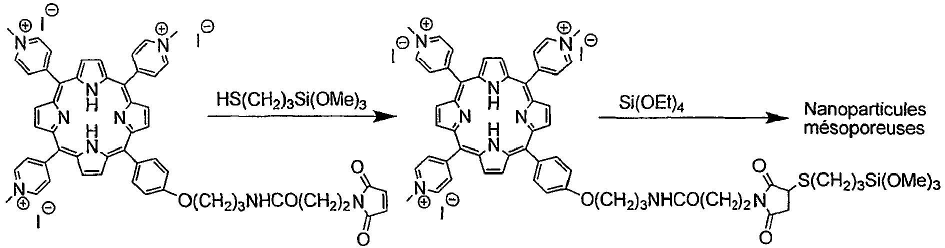

8 mg de la porphyrine **8** (6,3 10⁻³ mmol) sont dissous dans 1 mL de MeOH. 4,2 mg de mercaptopropyltriméthoxysilane (21,78 10⁻³ mmoles) sont additionnés et la réaction est maintenue à température ambiante pendant une nuit. 343 mg (0,9 10⁻³ mol) de bromure de cétyltriméthylammonium (CTAB) sont dissous dans 20 mL de soude 0,2M à 25°C. La solution précédemment préparée est ajoutée ainsi que le tétraéthoxysilane (1,75 mL, 0.8 10⁻² mmol). Après 40 secondes 128 mL d'eau déionisée sont ajoutés. La solution est maintenue pendant 6 minutes sous agitation puis rapidement neutralisée à pH 7 par l'addition de HCl 0,2 M (environ 25 mL). Les nanoparticules sont récupérées par centrifugation (20 minutes à 20000 tours-minute), remises en suspension dans EtOH aux ultrasons, centrifugées. Le tensioactif est extrait par traitement avec 15 mL de solution EtOH/HCl 12N (4/1) durant 2h à 60°C. Après centrifugation, l'opération est répétée deux fois, puis les nanoparticules sont remises en suspension dans l'eau et centrifugées jusqu'à obtenir un pH de l'ordre de 6 (6 fois).

### L'analyse IR confirme l'élimination du tensioactif

La TEM montre la présence d'un réseau hexagonal de mésopores avec un diamètre des nanoparticules de l'ordre de 150 nm.

La DLS montre un diamètre hydrodynamique de 311 nm.

L'analyse UV (EtOH) confirme la présence de la porphyrine encapsulée de manière covalente. On obtient une charge 4,43 µmol de porphyrine par g de nanoparticules.

La capacité des nanoparticules à générer de l'oxygène singulet est évaluée par phosphorescence de celui-ci après irradiation dans l'une des bandes d'absorption du photosensibilisateur et en particulier à 431 nm de 3 mg de nanoparticules dans 5 mL d'EtOH absolu. Le rendement quantique de formation de l'oxygène singulet est de 58%

### F- Synthèse de nanoparticules mésoporeuses encapsulant une porphyrine cationique - Particules OH 22 :

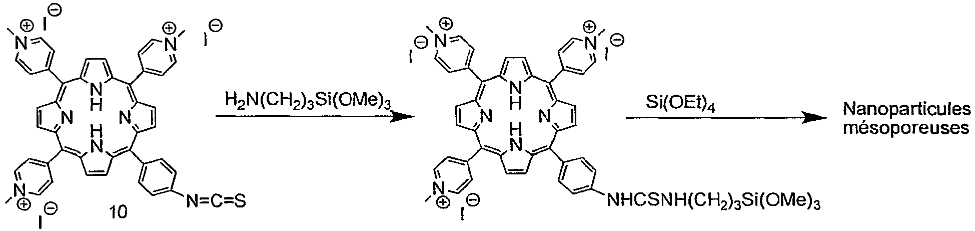

12 mg de la porphyrine 10 (1,1 10⁻² mmol) sont dissous dans 1 mL de MeOH. 4,88 mg d'aminopropyltriméthoxysilane (2,72 10⁻² mmoles) sont additionnés et la solution est maintenue à température ambiante pendant une nuit. 343 mg (0,9 10⁻³ mol) de bromure de cétyltriméthylammonium (CTAB) sont dissous dans 20 mL de soude 0,2M à 25°C. Le tétraéthoxysilane (1,75 mL, 0,8 10⁻² mmol) est ajouté goutte à goutte ainsi que la solution précédemment préparée. Après 40 secondes 128 mL d'eau déionisée sont ajoutés. La solution est maintenue pendant 6 minutes sous agitation puis rapidement neutralisée à pH 7 par l'addition de HCl 0,2 M (environ 25 mL). Les nanoparticules sont récupérées par centrifugation (20 minutes à 20000 tours-minute), remises en suspension dans EtOH aux ultrasons et centrifugées. Le tensioactif est extrait par traitement avec 15 mL de solution EtOH/HCl 12N (4/1) durant 2h à 60°C. Après centrifugation, l'opération est répétée deux fois, puis les nanoparticules sont remises en suspension dans l'eau et centrifugées jusqu'à obtenir un pH de l'ordre de 6 (6 fois).

### L'analyse IR confirme l'élimination du tensioactif.

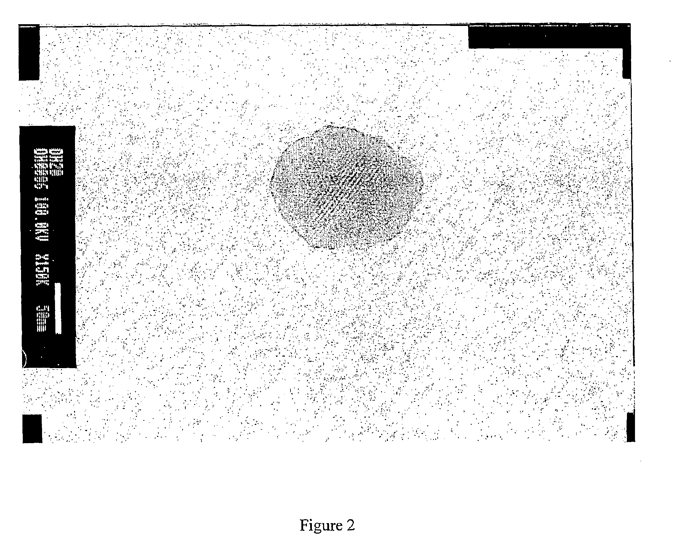

La TEM (Figure 2) montre la présence d'un réseau hexagonal de mésopores avec un diamètre des nanoparticules de l'ordre de 150 nm.

La DLS confirme le diamètre hydrodynamique de 153 nm.

L'analyse UV (EtOH) confirme la présence de la porphyrine encapsulée de manière covalente. On obtient une charge 0,97 µmol de porphyrine par g de nanoparticules.

La capacité des nanoparticules à générer de l'oxygène singulet est évaluée par phosphorescence de celui-ci après irradiation dans l'une des bandes d'absorption du photosensibilisateur et en particulier à 431 nm de 3 mg de nanoparticules dans 5 mL d'EtOH absolu. Le rendement quantique de formation de l'oxygène singulet est de 60%

### G- Greffage de l'α-mannose en surface des nanoparticules :

### - Particules DB016 :

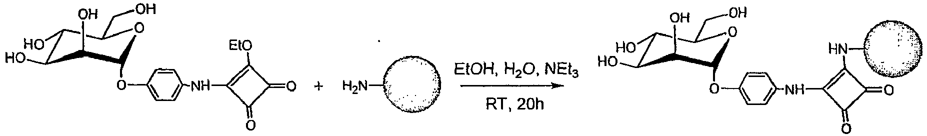

100 mg de nanoparticules DB 015 aminées préparées au § B ci-dessus sont dispersées aux ultrasons pendant 10 minutes dans 5 mL d'EtOH. 59 mg (0,15 mmol) d'aryl squarate d'α-mannose sont dissous dans 5 mL d'un mélange EtOH-H₂O (50-50). La solution est ajoutée à la précédente goutte à goutte. 500 µL de triéthylamine sont ajoutés et la suspension est agitée pendant 18h. Après centrifugation les nanoparticules sont redispersées dans l'eau et centrifugées (3 cycles). Puis elles sont redispersées dans EtOH et centrifugées (deux cycles).

L'analyse DLS indique un diamètre hydrodynamique de 173 nm.

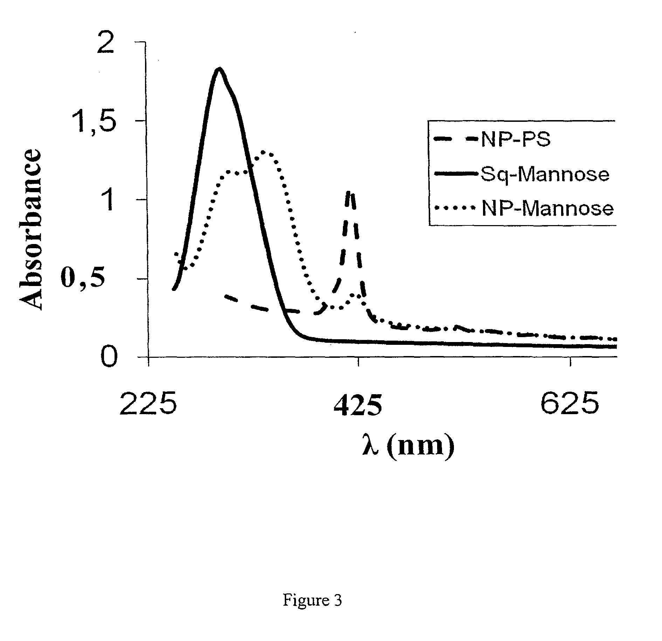

L'analyse UV confirme la présence de la porphyrine et du noyau aromatique-squarate (Figure 3).

### - Nanoparticules DB 021 :

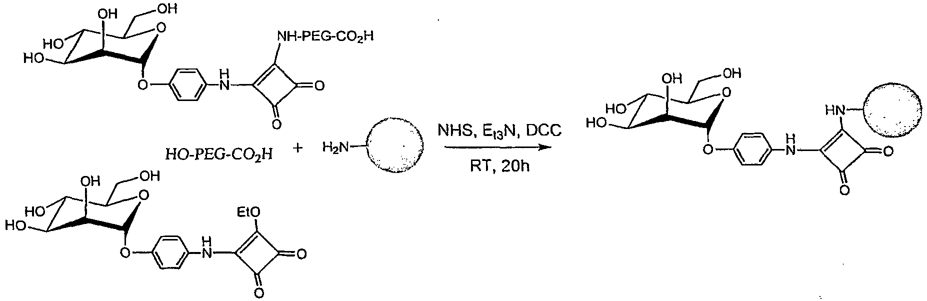

250 mg (7,35 10⁻⁵ mol) de H₂N-PEG-CO₂H (masse molaire 3400) sont dissous dans 1,25 mL d'eau distillée. 2 équivalents (0,0148 mol) de triéthylamine sont ajoutés. 81 mg (2,5 équivalents) de squarate phényl mannose sont dissous dans 1 mL d'eau. Cette solution est ajoutée goutte à goutte à la précédente. La réaction est maintenue à température ambiante durant 15h. Le DMF est évaporé puis le PEG est cristallisé dans l'éther qui est évaporé.

66 mg (2.10⁻⁵ mol) de produit précédent, 256 mg (9.10⁻⁵ mol) de HO₂C-PEG-OH, sont dissous dans 10 mL de DMF. 21 µl d'Et₃N, 15 mg de NHS (N-hydroxysuccinimide) 30 mg de DCC (dicyclohexylcarbodiimide) sont ajoutés. La réaction est maintenue une nuit à température ambiante, puis 200 mg de nanoparticules DB 019 sont ajoutées. Après 20h, la solution est centrifugée, les nanoparticules remises en suspension dans le DMF aux ultrasons puis la solution est centrifugée à nouveau. Le lavage au DMF est répété une fois, puis les nanoparticules sont lavées trois fois à l'eau, deux fois à EtOH.

On obtient 178 mg de nanoparticules DB 021.

### H- Greffage de l'α-mannose-6-carboxylate en surface des nanoparticules (Particules DB 054) :

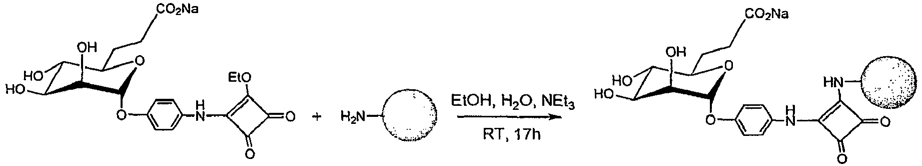

56 mg de nanoparticules aminées DB 015 sont redispersées aux ultrasons pendant 10 minutes dans 5 mL d'EtOH. 25 mg (0,055 mmol) de squarate α-mannose-6carboxylate sont dissous dans 5 mL d'un mélange EtOH-H₂O (3/2). La solution est ajoutée à la précédente goutte à goutte. 400 µL de triéthylamine sont ajoutés et la suspension agitée pendant 17h. Après centrifugation les nanoparticules sont redispersées dans l'eau et centrifugées (3 cycles), puis redispersées dans EtOH et centrifugées (trois cycles).

On obtient 33 mg de nanoparticules DB 054.

### III- Activité biologique :

Les activités en thérapie photodynamique (PDT) des différentes nanoparticules non fonctionnalisées ou fonctionnalisées par une biomolécule sont évaluées dans différentes lignées cellulaires. L'efficacité cytotoxique des nanoparticules internalisées dans des cellules cancéreuses est testée après excitation monophotonique des porphyrines encapsulées. L'efficacité de la PDT est montrée par la relative innocuité des particules non irradiées. La spécificité du ciblage et de l'endocytose des nanoparticules est montrée par co-incubation de la nanoparticule fonctionnalisée et de la biomolécule correspondante.

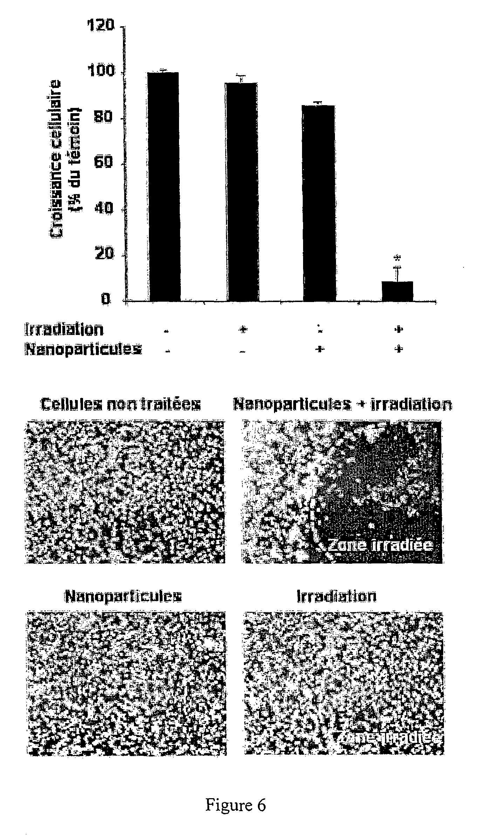

### Exemple 1 : Activité PDT des nanoparticules OH21 sur les cellules de cancer du sein MDA-MB-231 (Figure 6)

*Conditions expérimentales* : les cellules de cancer du sein MDA-MB-231, maintenues en culture dans du milieu Dulbecco's modified Eagle's medium additionné de 10% de FCS, dans une atmosphère humide à 37°C et 5% de CO₂, sont ensemencées dans des plaques 96 puits à une densité de 30 000 cellules par puits, dans 100 µl de milieu. Les cellules de cancer du sein MDA-MB-231 ayant des capacités de phagocytose, sont incubées pendant 24 h avec 20 µg/ml de nanoparticules et irradiées pendant 40 min par un laser à 650 nm (puissance 2 à 10 mW/cm²). Deux jours après l'irradiation, les cellules vivantes sont quantifiées par le test enzymatique du MTT (bromure de 3-(4,5-diméthylthiazol-2-yl)-2,5-diphényltétrazolium, Sigma).

*Résultats :* l'efficacité de la thérapie photodynamique se traduit par une inhibition de la croissance cellulaire de 92 %. Les absences de toxicité des nanoparticules non irradiées et de l'irradiation en absence de nanoparticules démontrent la spécificité de la thérapie photodynamique avec ces nanoparticules. Les cellules sont visualisées par un marquage fluorescent des noyaux au DAPI. Les clichés obtenus montrent la spécificité de la thérapie photodynamique à l'aide des nanoparticules de l'invention. La mort cellulaire est évidente et de façon exclusive dans la zone irradiée et en présence de nanoparticules.

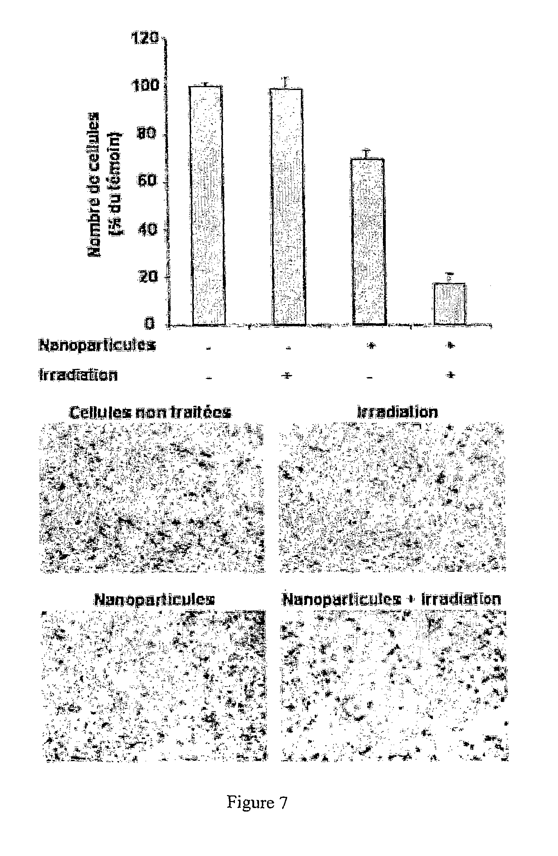

### Exemple 2 : Activité PDT des nanoparticules OH21 sur les cellules de cancer de l'ovaire POE14 (Figure 7).

*Conditions expérimentales :* les cellules POE14 sont maintenues en culture comme décrit dans l'Exemple 1, incubées pendant 24 h avec une concentration de nanoparticules réduite à 10 µg/ml et irradiées comme décrit dans l'Exemple 1. Deux jours après irradiation, l'ajout dans le milieu de MTT permet de quantifier le nombre de cellules vivantes.

*Résultats :* l'irradiation des nanoparticules incubées avec les cellules POE14 induit une mort cellulaire de 82 % par rapport aux cellules témoins ou seulement irradiées. Dans ce type cellulaire, les nanoparticules non irradiées induisent une diminution du nombre de cellules vivantes d'environ 30 %. Cette toxicité démontre la nécessité d'un ciblage spécifique.

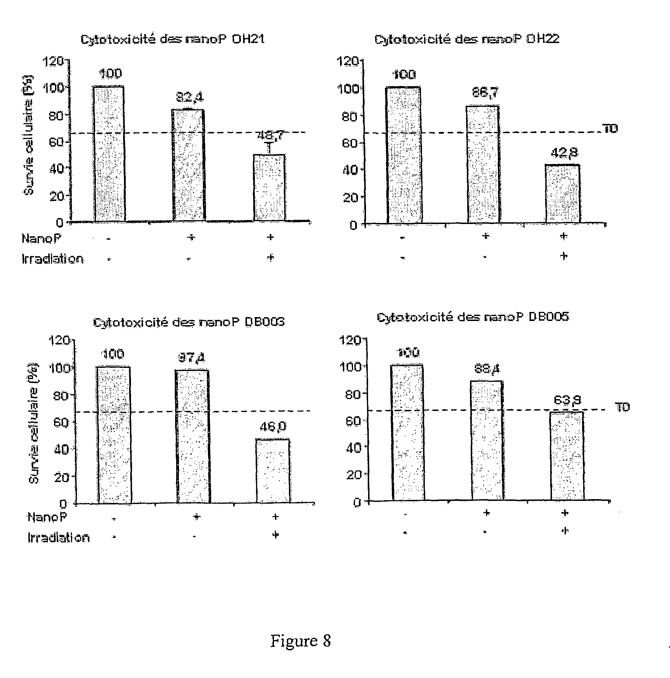

### Exemple 3 : Activité PDT des nanoparticules cationiques OH21, OH22 et anioniques DB003, DB005 sur les cellules de cancer du sein MDA-MB-231 (Figure 8).

*Conditions expérimentales :* identiques à l'Exemple 1.

*Résultats* : Les résultats obtenus permettent de comparer l'efficacité des nanoparticules contenant soit des porphyrines anioniques, soit des porphyrines cationiques. La figure 8 montre que l'ensemble des nanoparticules irradiées testées inhibe totalement la croissance des cellules MDA-MB-231 comparée à la valeur T0 qui correspond à l'ensemencement cellulaire initial avant irradiation. De plus, les irradiations des nanoparticules OH21 et OH22 qui contiennent des porphyrines cationiques induisent une mort cellulaire de 51,3 % et 57,2 % respectivement par rapport au témoin (cellules non traitées). Les nanoparticules DB003 et DB005 contenant des porphyrines anioniques induisent après irradiation une mort cellulaire de 37,1 % et 54 % respectivement. La cytotoxicité (faible mais significative) de certaines nanoparticules non irradiées démontre les avantages d'un ciblage spécifique provenant du mannose.

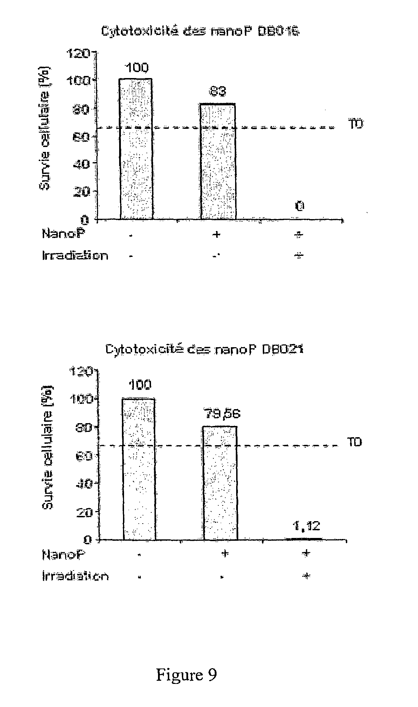

### Exemple 4: Activité PDT des nanoparticules anioniques fonctionnalisées par du mannose sur les cellules de cancer du sein MDA-MB-231 (Figure 9).

*Conditions expérimentales :* identiques à l'Exemple 1.

*Résultats :* Les résultats obtenus permettent de comparer l'efficacité des nanoparticules contenant des porphyrines anioniques, recouvertes ou non de mannose. Les nanoparticules DB016 et DB021 à la surface desquelles ont été greffés des résidus mannose, induisent une mort cellulaire de 100 % et 99 %, respectivement. Ces résultats mettent en évidence l'augmentation de l'efficacité cytotoxique des nanoparticules par le ciblage provenant du mannose.

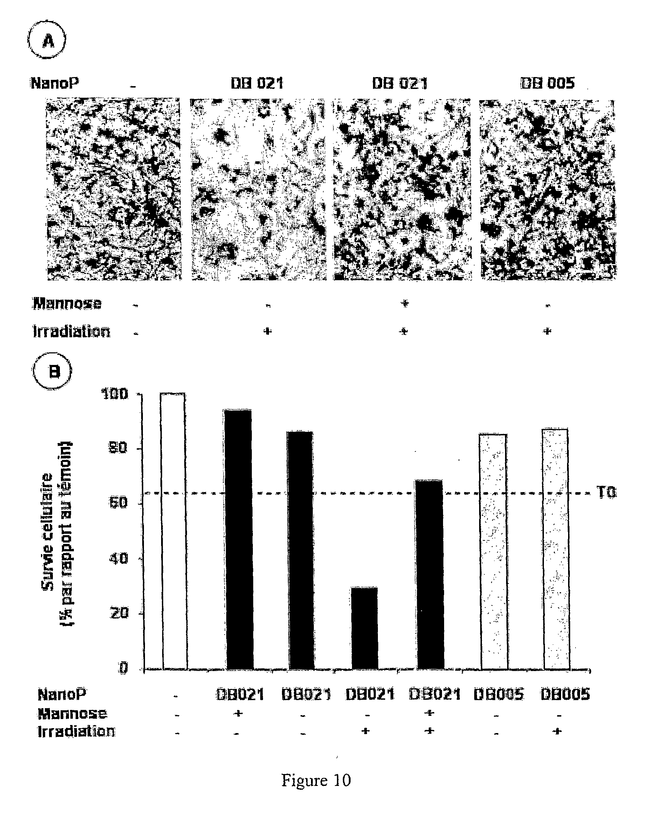

### Exemple 5 : Comparaison des activités PDT des nanoparticules anioniques fonctionnalisées par du mannose ou non fonctionnalisées sur les cellules de cancer du sein MDA-MB-231 en présence ou non d'un excès de mannose (Figure 10).

*Conditions expérimentales :* Les cellules sont incubées pendant 6 heures dans du milieu supplémenté ou non de mannose (10⁻² M), en présence ou en absence 20 µg/ml de nanoparticules (DB021 ou DB005). Après 6 h d'incubation, les nanoparticules du milieu sont éliminées et les cellules sont rincées 2 fois puis mises en culture dans 100 µl de milieu frais. Les cellules sont alors soumises à une irradiation au laser de longueur d'onde de 630-680 nm, avec une puissance de 2 à 10 mW/cm², pendant 40 min. 48 h après irradiation, les cellules vivantes sont mises en évidence (A) et quantifiées (B) par le MTT.

*Résultats :* la figure 10A montre le niveau témoin de l'accumulation du MTT dans les cellules non traitées. L'incubation des cellules avec les nanoparticules DB021 et soumises à l'irradiation conduit à une mort cellulaire. Ce phénomène est bloqué par la préincubation avec un excès de mannose. Les nanoparticules DB005 irradiées n'induisent pas de mort cellulaire. La quantification des cellules vivantes (Figure 10B) confirme ces observations. La cytotoxicité due aux nanoparticules DB021 incubées pendant 6 h n'est que de 2 à 10 % (± mannose) alors que l'irradiation des nanoparticules DB021 induit 70 % de mort cellulaire. L'ajout d'un excès de mannose stoppe en grande partie cet effet ce qui démontre la spécificité de l'internalisation. D'autre part, les nanoparticules DB005 irradiées ou non n'induisent que 5 à 10 % de mort cellulaire.

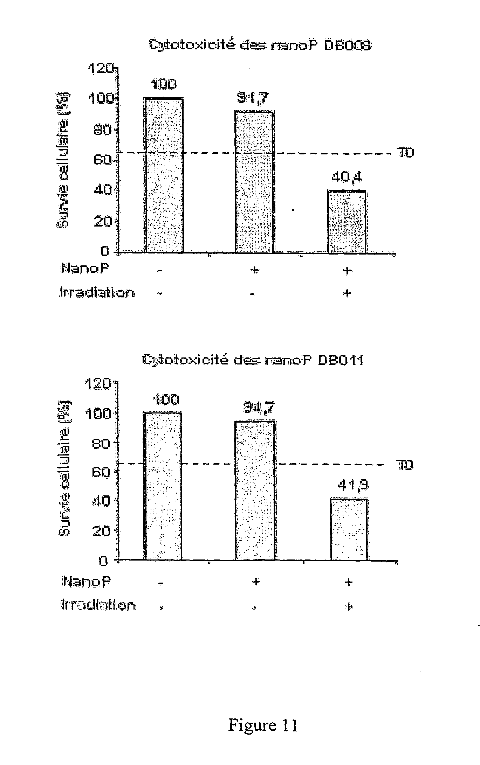

### Exemple 6 : Activité PDT des nanoparticules de silicalite DB008 et DB011 sur les cellules de cancer du sein MDA-MB-231 (Figure 11).

*Conditions expérimentales:* identiques à l'Exemple 1.

*Résultats :* Les résultats obtenus montrent que les nanoparticules de silicalites DB008 et DB011 présentent une faible cytotoxicité de 8,3% et 5,3% lorsqu'elles ne sont pas irradiées. L'irradiation des cellules incubées avec ces nanoparticules (pendant 24 h) induit une inhibition totale de la croissance et une mort cellulaire d'environ 60 %.

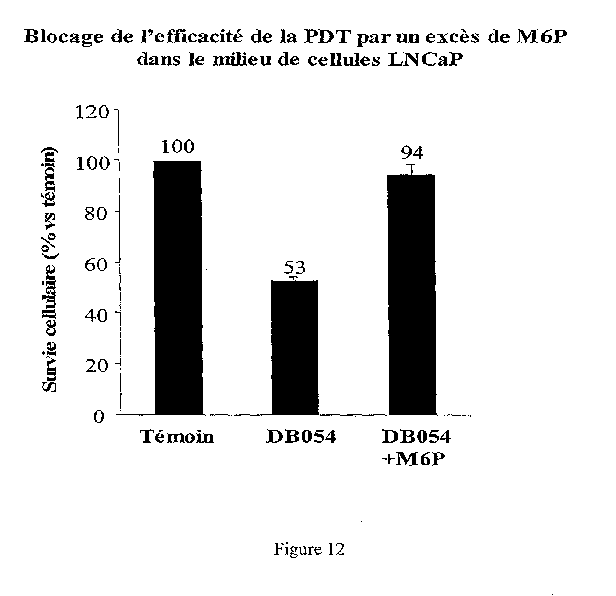

### Exemple 7 : Activité PDT des nanoparticules DB054 sur les cellules de cancer de la prostate LNCaP : spécificité de l'internalisation de DB054 (Figure 12).

*Conditions expérimentales :* Des cellules humaines de cancer de la prostate (LNCaP) sont incubées pendant 1 h avec les nanoparticules DB054 dans un milieu sans sérum à 37°C, en présence ou en absence d'un excès de M6P puis soumises à une irradiation laser (660 nm, 6-7 mW/cm², 40 min). Le pourcentage de cellules vivantes est déterminé à l'aide du test MTS (Promega). Les représentations graphiques correspondent à la moyenne de 3 expériences indépendantes.

*Résultats :* Les résultats obtenus montrent que les nanoparticules DB054 induisent une mort cellulaire de 47% après une heure de traitement. Cet effet est bloqué par l'ajout dans le milieu d'un excès de 10 mM M6P, ce qui démontre une internalisation des nanoparticules DB054 par le récepteur membranaire du M6P.

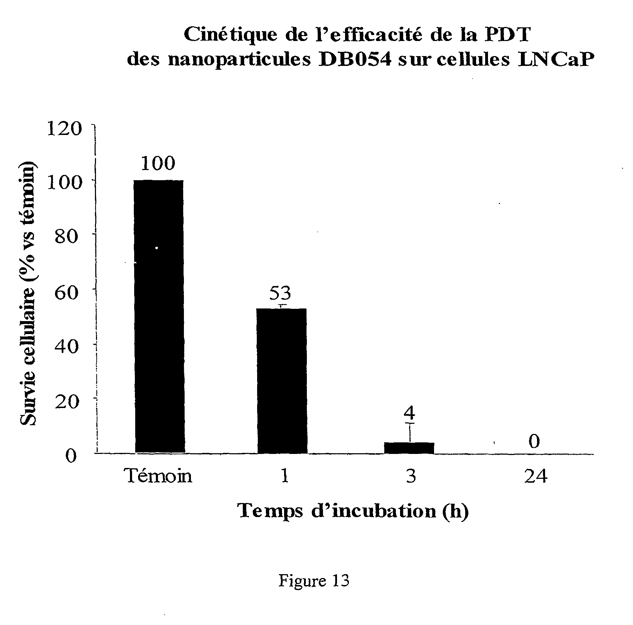

### Exemple 8 : Activité PDT des nanoparticules DB054 sur les cellules de cancer de la prostate LNCaP : variation de l'efficacité dans le temps de DB054 (Figure 13).

*Conditions expérimentales :* Des cellules humaines de cancer de la prostate (LNCaP) sont incubées pendant des durées différentes (1 h, 3 h, 24 h) avec les nanoparticules DB054 puis soumises à une irradiation laser (660 nm, 6-7 mW/cm², 40 min). Le pourcentage de cellules vivantes est déterminé à l'aide du test MTS (Promega). Les représentations graphiques correspondent à la moyenne de 3 expériences indépendantes.

*Résultats :* Les résultats obtenus montrent que les nanoparticules DB054 induisent respectivement 47%, 96% et 100% de cytotoxicité après 1 h, 3 h et 24 h de traitement. Ces résultats mettent en évidence la rapidité d'action des nanoparticules.

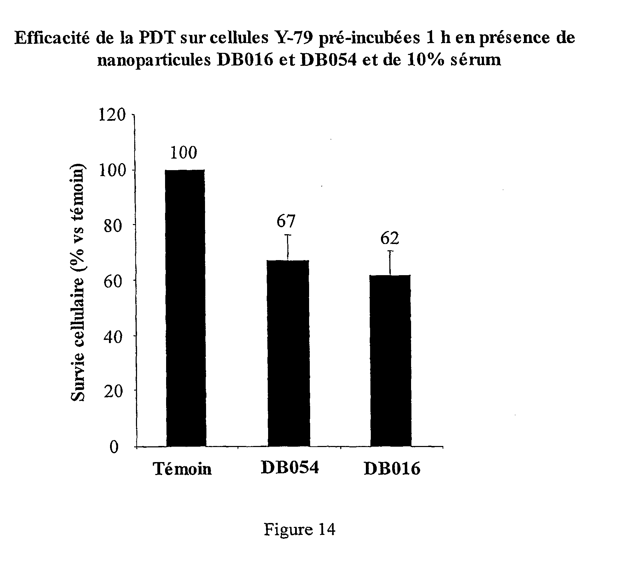

### Exemple 9 : Activité PDT des nanoparticules DB016 et DB054 sur les cellules humaines de rétinoblastome (Figure 14).

*Conditions expérimentales :* Des cellules humaines de rétinoblastome (Y-79) sont incubées pendant 1 h avec les nanoparticules DB054 ou DB016 dans un milieu complet à 37°C puis soumises à une irradiation laser (660 nm, 6-7 mW/cm², 40 min). Le pourcentage de cellules vivantes est déterminé à l'aide du test MTS (Promega). Les représentations graphiques correspondent à la moyenne de 3 expériences indépendantes.

*Résultats :* Les résultats obtenus montrent que les nanoparticules DB054 et DB016 induisent respectivement 33% et 38% de cytotoxicité cellulaire. Ceci démontre que les nanoparticules fonctionnalisées par le mannose-6-phosphotate ou par le mannose peuvent être internalisées de façon efficace via différentes lectines présentes à la surface des cellules Y-79.

## Revendications

1. Molécule répondant à la formule (I) ci-dessous : 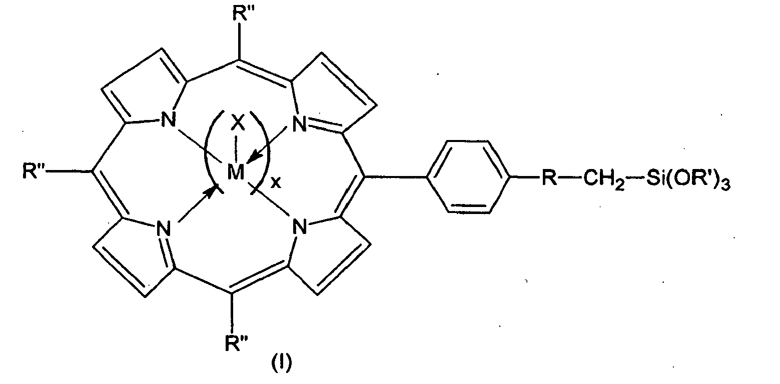 dans laquelle :
x représente un entier choisi parmi : 0 et 1,
M représente un atome de métal choisi parmi les métaux de transition,
lorsque x représente 0, (M-X) est remplacé par deux atomes d'hydrogène,
X représente un groupement choisi parmi : un halogénure, un anion d'un acide carboxylique pharmaceutiquement acceptable,
R représente un groupement choisi parmi :
une chaîne alkyle en C₁-C₁₅, éventuellement interrompue par un ou plusieurs groupements choisis parmi : un éther (-O-), une amine (-NH-), un thioéther (-S-), une cétone (-CO-), un ester (-CO-O-), un amide (-CO-NH-), une urée (-NH-CO-NH-), une thiourée (-NH-CS-NH-), un oxycarbonyl (-O-CO-O-), un carbamate (-NH-CO-O-), 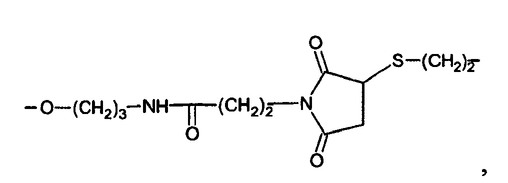
R' représente un groupement choisi parmi : un alkyle en C₁-C₆, un phényle, un benzyle,
R" représente un groupement choisi parmi : 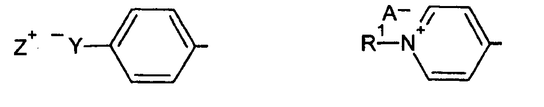
et Z⁺ représente un cation organique ou minéral pharmaceutiquement acceptable,
Y⁻ représente un groupement qui peut être choisi parmi : -COO⁻, -SO₃⁻,
A⁻ représente un anion qui peut être choisi parmi : un halogénure, un anion d'un acide carboxylique pharmaceutiquement acceptable,
R¹ représente un alkyle linéaire, ramifiée ou cyclique en C₁ à C₁₀.

2. Molécule selon la revendication 1, dans laquelle
M représente un atome de métal choisi parmi : parmi : Zn, Pt, Pd, Mn, Gd, Ni, Cr, Ru.
X représente un groupement choisi parmi : Cl⁻, Br⁻, I⁻, acétate, propionate, butyrate, ascorbate, benzoate, cinnamate, citrate, fumarate, glycolate, malonate, tartrate, malate, maléate, mandélate, tosylate,
R représente un groupement choisi parmi :
une chaîne alkyle en C₁-C₁₀, éventuellement interrompue par un ou plusieurs groupements choisis parmi : un éther (-O-), une amine (-NH-), un thioéther (-S-), une cétone (-CO-), un ester (-CO-O-), un amide (-CO-NH-), une urée (-NH-CO-NH-), une thiourée (-NH-CS-NH-), un oxycarbonyl (-O-CO-O-), un carbamate (-NH-CO-O-),
R' représente un groupement choisi parmi : un alkyle en C₁-C₃,
R¹ représente un groupement choisi parmi : un alkyle en C₁-C₃,
Z⁺ représente un cation qui peut être choisi parmi : K⁺, Li⁺, Na⁺, NH₄⁺,
A⁻ représente un anion qui peut être choisi parmi : Cl⁻, Br⁻, I⁻, acétate, propionate, butyrate, ascorbate, benzoate, cinnamate, citrate, fumarate, glycolate, malonate, tartrate, malate, maléate, mandélate, tosylate..

3. Molécule selon la revendication 2, dans laquelle
X représente un groupement choisi parmi : Cl⁻, Br⁻, I⁻, acétate, tosylate,
R est choisi parmi : 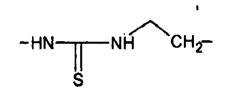 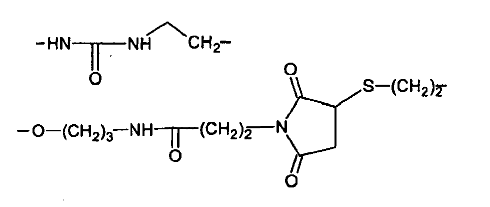
R' représente un groupement choisi parmi : un méthyle, un éthyle,
R¹ représente un méthyle,
A⁻ représente un anion qui peut être choisi parmi : Cl⁻, Br⁻, I⁻, acétate, tosylate.

4. Molécule selon l'une quelconque des revendications 1 à 3, qui est choisie parmi :

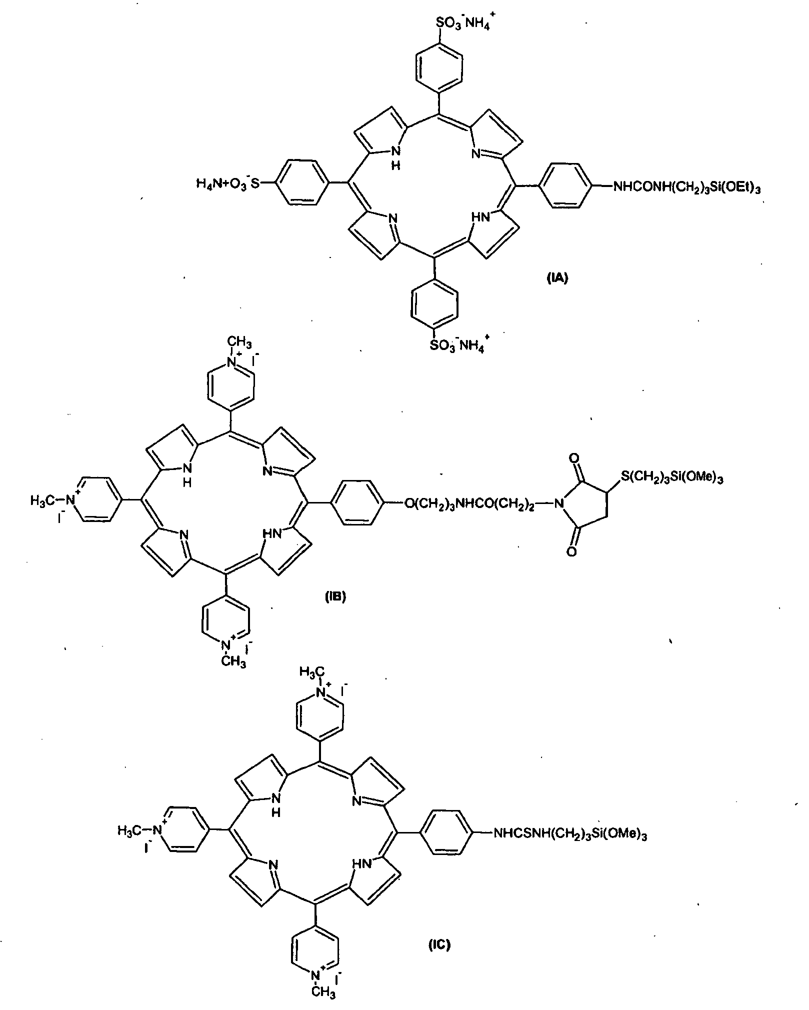

5. Composition de nanoparticules à base de silice comportant au moins un photosensibilisateur répondant à la formule (I) selon l'une quelconque des revendications 1 à 4.

6. Composition selon la revendication 5 dans laquelle les nanoparticules sont à porosité organisée.

7. Composition selon la revendication 6, dans laquelle les nanoparticules de silice sont mésoporeuses.

8. Composition selon la revendication 7 dans laquelle les nanoparticules de silice mésoporeuse ont une taille de particules allant de 80 à 400 nm de diamètre, une surface spécifique allant de 800 à 1000 m²/g et des pores de taille allant de 2 à 6 nm.

9. Composition selon la revendication 6, dans laquelle les nanoparticules de silice sont microporeuses.

10. Composition selon la revendication 9, dans laquelle les nanoparticules ont un diamètre compris entre 40 et 80 nm, une surface spécifique allant de 100 à 450 m²/g et des diamètres de pores allant de 2 à 20 Å.

11. Composition selon l'une quelconque des revendications 5 à 10, qui comporte un greffage sur la surface des nanoparticules par des molécules de ciblage spécifiques des tissus néoplasiques.

12. Composition selon la revendication 11, dans laquelle la molécule de ciblage est choisie parmi les dérivés de sucre, en particulier le mannose.

13. Composition selon l'une quelconque des revendications 5 à 12, pour son utilisation comme médicament.

14. Composition selon la revendication 13, pour son utilisation pour le traitement et/ou la prévention et/ou la détection d'un cancer.

## Patentansprüche

1. Molekül mit der unten angegebenen Formel (I): 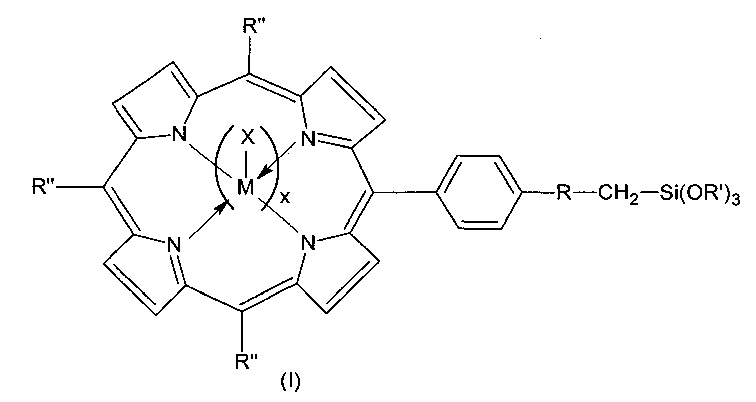 bei der:
x eine zwischen 0 und 1 ausgewählte ganze Zahl ist,
M ein Metallatom ist, das aus den Übergangsmetallen ausgewählt ist,
(M-X) durch zwei Wasserstoffatome ersetzt ist, wenn x gleich 0 ist,
X eine aus einem Halogenid und einem Anion einer pharmazeutisch akzeptablen Carbonsäure ausgewählte Gruppe ist,
R eine Gruppe ist, die ausgewählt ist aus:
einer C₁-C₁₅-Alkylkette, die gegebenenfalls durch eine oder mehrere Gruppen unterbrochen ist, die aus einem Ether (-O-), einem Amin (-NH-), einem Thioether (-S-), einem Keton (-CO-), einem Ester (-CO-O-), einem Amid (-CO-NH-), einem Harnstoff (-NH-CO-NH-), einem Thioharnstoff (-NH-CS-NH-), einem Oxycarbonyl (-O-CO-O-), einem Carbamat (-NH-CO-O-) und 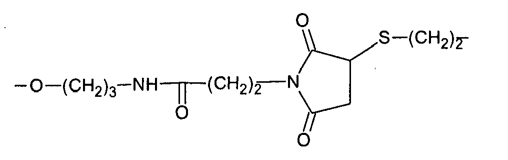 ausgewählt sind,
R' eine aus einem C₁-C₆-Alkyl, einem Phenyl und einem Benzyl ausgewählte Gruppe ist,
R" eine Gruppe ist, die ausgewählt ist aus: 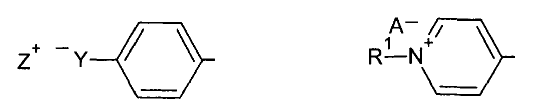
und Z⁺ ein pharmazeutisch akzeptables organisches oder mineralisches Kation ist,
Y⁻ eine Gruppe ist, die aus -COO- und -SO₃ ausgewählt sein kann,
A⁻ ein Anion ist, das aus einem Halogenid und einem Anion einer pharmazeutisch akzeptablen Carbonsäure ausgewählt sein kann,
R¹ ein lineares, verzweigtes oder zyklisches C₁ bis C₁₀-Alkyl ist.

2. Molekül nach Anspruch 1, bei dem
M ein aus Zn, Pt, Pd, Mn, Gd, Ni, Cr und Ru ausgewähltes Metallatom ist,
X eine aus Cl-, Br⁻, I⁻, Acetat, Propionat, Butyrat, Ascorbat, Benzoat, Cinnamat, Zitrat, Fumarat, Glycolat, Malonat, Tartrat, Malat, Maleat, Mandelat und Tosylat ausgewählte Gruppe ist,
R eine Gruppe ist, die ausgewählt ist aus:
einer C₁-C₁₀-Alkylkette, die gegebenenfalls durch eine oder mehrere Gruppen unterbrochen ist, die aus einem Ether (-O-), einem Amin (-NH-), einem Thioether (-S-), einem Keton (-CO-), einem Ester (-CO-O-), einem Amid (-CO-NH-), einem Harnstoff (-NH-CO-NH-), einem Thioharnstoff (-NH-CS-NH-), einem Oxycarbonyl (-O-CO-O-) und einem Carbamat (-NH-CO-O-) ausgewählt sind,
R' eine aus einem C₁-C₃-Alkyl ausgewählte Gruppe ist,
R¹ eine aus einem C₁-C₃-Alkyl ausgewählte Gruppe ist,
Z⁺ ein Kation ist, das aus K⁺, Li⁺, Na⁺ und NH₄⁺ ausgewählt sein kann,
A⁻ ein Anion ist, das aus Cl⁻, Br⁻, I⁻, Acetat, Propionat, Butyrat, Ascorbat, Benzoat, Cinnamat, Zitrat, Fumarat, Glycolat, Malonat, Tartrat, Malat, Maleat, Mandelat und Tosylat ausgewählt sein kann.

3. Molekül nach Anspruch 2, bei dem
X eine aus Cl⁻, Br⁻, I⁻, Acetat und Tosylat ausgewählte Gruppe ist,
R ausgewählt ist aus: 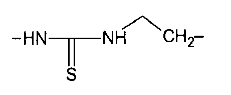 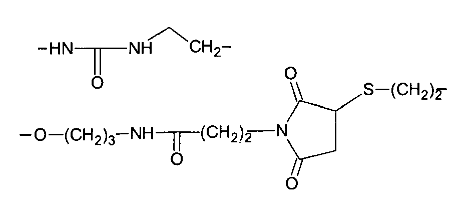
R' eine aus Methyl und Ethyl ausgewählte Gruppe ist,
R¹ Methyl ist,
A⁻ ein Anion ist, das aus Cl⁻, Br⁻, I⁻, Acetat und Tosylat ausgewählt sein kann.

4. Molekül nach einem der Ansprüche 1 bis 3, das ausgewählt ist aus:

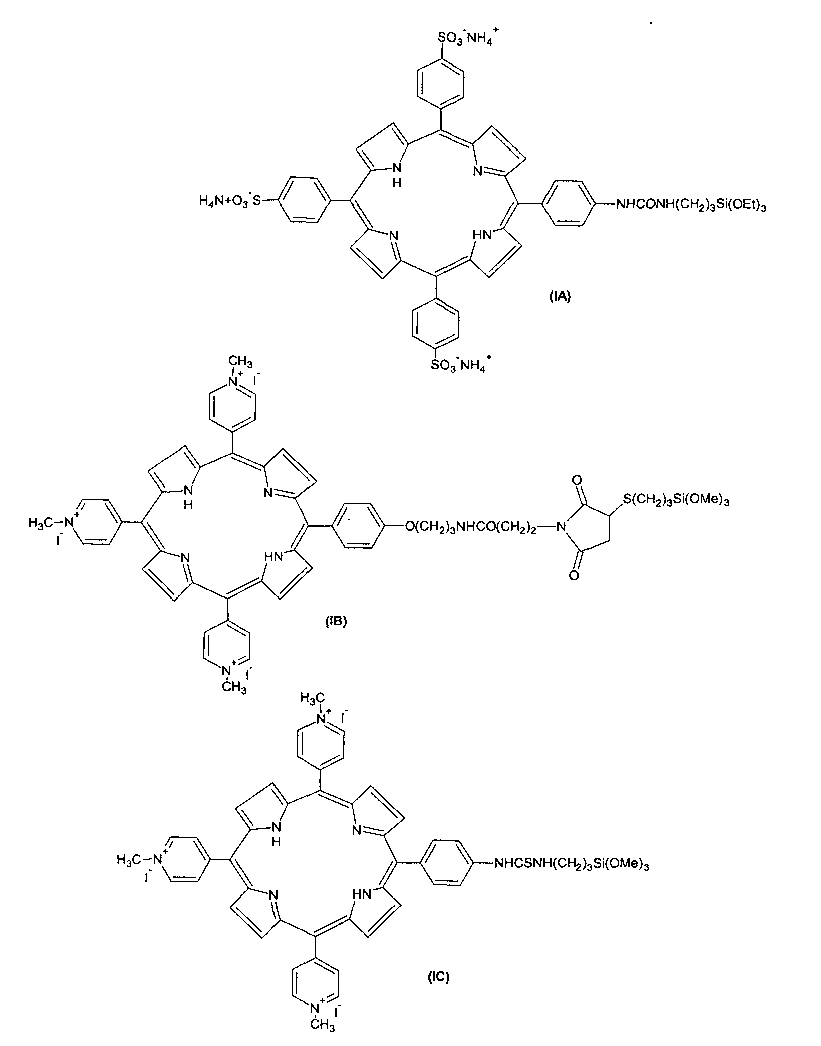

5. Nanopartikelzusammensetzung auf Siliciumdioxidbasis, die mindestens einen Fotosensibilisator mit der Formel (I) nach einem der Ansprüche 1 bis 4 aufweist.

6. Zusammensetzung nach Anspruch 5, bei der die Nanopartikel eine organisierte Porosität aufweisen.

7. Zusammensetzung nach Anspruch 6, bei der die Siliciumdioxidnanopartikel mesoporös sind.

8. Zusammensetzung nach Anspruch 7, bei der die mesoporösen Siliciumdioxidnanopartikel eine Partikelgröße mit einem Durchmesser von 80 bis 400 nm, eine spezifische Oberfläche von 800 bis 1000 m²/g und Poren mit einer Größe von 2 bis 6 nm aufweisen.

9. Zusammensetzung nach Anspruch 6, bei der die Siliciumdioxidnanopartikel mikroporös sind.

10. Zusammensetzung nach Anspruch 9, bei der die Nanopartikel einen Durchmesser von zwischen 40 und 80 nm, eine spezifische Oberfläche von 100 bis 450 m²/g und Porendurchmesser von 2 bis 20 Å aufweisen.

11. Zusammensetzung nach einem der Ansprüche 5 bis 10, die auf der Oberfläche der Nanopartikel eine Propfung durch Targeting-Moleküle aufweist, die für neoplastische Gewebe spezifisch sind.

12. Zusammensetzung nach Anspruch 11, bei der das Targeting-Molekül aus Zuckerderivaten, insbesondere Mannose ausgewählt ist.

13. Zusammensetzung nach einem der Ansprüche 5 bis 12 zur Verwendung als Arzneimittel.

14. Zusammensetzung nach Anspruch 13 zur Verwendung bei der Behandlung und/oder Vorbeugung und/oder Erkennung von Krebs.

## Claims

1. Molecule having the general formula (I) given below: 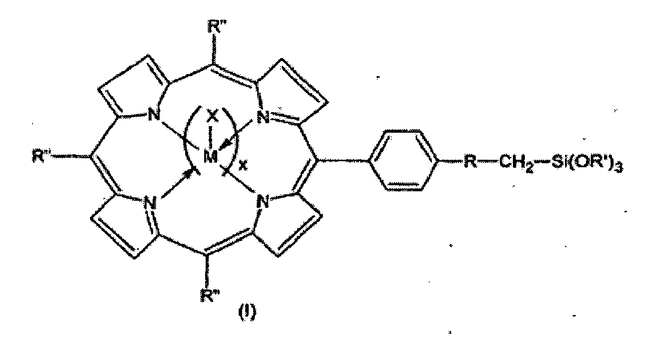 in which:
x is an integer selected from 0 and 1,
M is an atom of a metal selected from the transition metals,
when x is 0, (M-X) is replaced by two hydrogen atoms,
X is a group selected from: a halide, an anion of a pharmaceutically acceptable carboxylic acid,
R is a group selected from: a C₁-C₁₅ alkyl chain, possibly interrupted by one or more groups selected from: an ether (-O-), an amine (-NH-), a thioether (-S-), a ketone (-CO-), an ester (-CO-O-), an amide (-CO-NH-), a urea (-NH-CO-NH-), a thiourea (-NH-CS-NH-), an oxycarbonyl (-O-CO-O-), a carbamate (-NH-CO-O-), 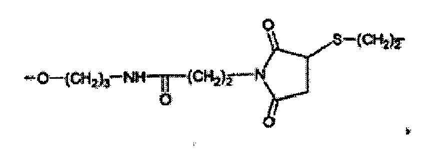
R' is a group selected from: a C₁-C₆ alkyl, a phenyl, a benzyl,
R" is a group selected from:
Z⁺ 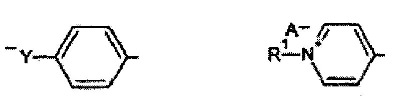
and Z⁺ is a pharmaceutically acceptable organic or mineral cation,
Y⁻ is a group which may be selected from: -COO⁻, -SO₃⁻,
A⁻ is an anion which may be selected from: a halide, an anion of a pharmaceutically acceptable carboxylic acid,
R¹ is a straight-chain, branching or cyclic C₁ to C₁₀ alkyl.

2. Molecule according to claim 1, in which
M is an atom of a metal selected from: Zn, Pt, Pd, Mn, Gd, Ni, Cr, Ru,
X is a group selected from: Cl⁻, Br⁻, I⁻, acetate, propionate, butyrate, ascorbate, benzoate, cinnamate, citrate, fumarate, glycolate, malonate, tartrate, malate, maleate, mandelate, tosylate,
R is a group selected from: a C₁-C₁₀ alkyl chain, possibly interrupted by one or more groups selected from: an ether (-O-), an amine (-NH-), a thioether (-S-), a ketone (-CO-), an ester (-CO-O-), an amide (-CO-NH-), a urea (-NH-CO-NH-), a thiourea (-NH-CS-NH-), an oxycarbonyl (-O-CO-O-), a carbamate (-NH-CO-O-),
R' is a group selected from: a C₁-C₃ alkyl,
R¹ is a group selected from: a C₁-C₃ alkyl,
Z⁺ is a cation which may be selected from: K⁺, Li⁺, Na⁺, NH₄⁺,
A⁻ is an anion which may be selected from: Cl⁻, Br⁻, I⁻, acetate, propionate, butyrate, ascorbate, benzoate, cinnamate, citrate, fumarate, glycolate, malonate, tartrate, malate, maleate, mandelate, tosylate.

3. Molecule according to claim 2, in which
X is a group selected from: Cl⁻, Br⁻, I⁻, acetate, tosylate,
R is selected from: 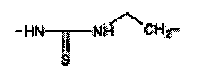 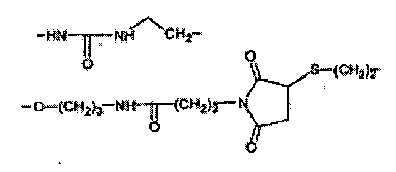
R' is a group selected from: a methyl, an ethyl,
R¹ is a methyl,
A⁻ is an anion which may be selected from: Cl⁻, Br⁻, I⁻, acetate, tosylate.

4. Molecule according to any one of claims 1 to 3 which is selected from:

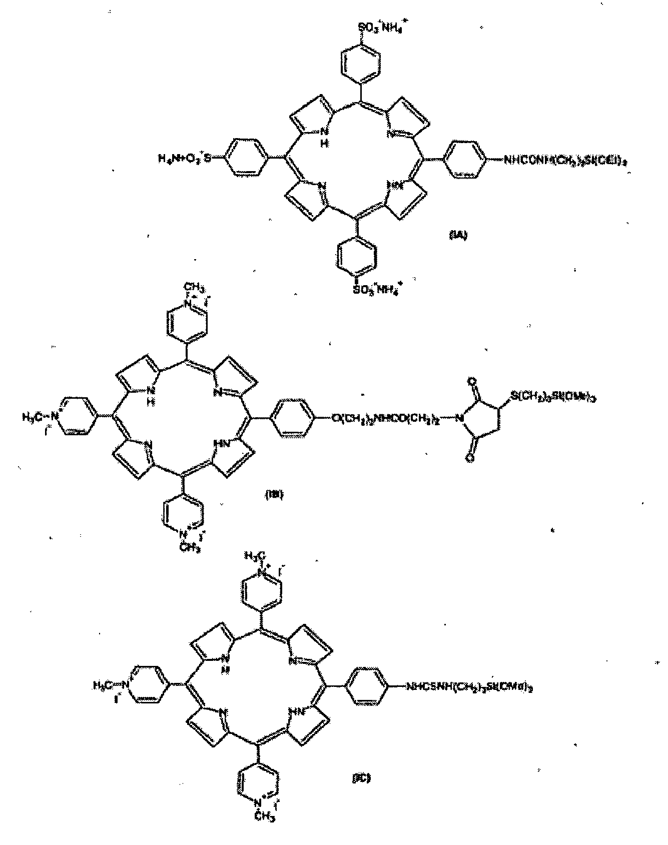

5. Composition of silica-based nanoparticles comprising at least one photosensitiser having the general formula (I) according to any one of claims 1 to 4.

6. Composition according to claim 5 in which the nanoparticles have an organised porosity.

7. Composition according to claim 6 in which the silica nanoparticles are mesoporous.

8. Composition according to claim 7 in which the nanoparticles of mesoporous silica have a particle size ranging from 80 to 400 nm in diameter, a specific surface area ranging from 800 to 1000 m²/g and pores with a size ranging from 2 to 6 nm.

9. Composition according to claim 6 in which the silica nanoparticles are microporous.

10. Composition according to claim 9 in which the nanoparticles have a diameter of between 40 and 80 nm, a specific surface area ranging from 100 to 450 m²/g and pore diameters ranging from 2 to 20 Å.

11. Composition according to any one of claims 5 to 10, which comprises grafting to the surface of the nanoparticles of molecules for screening specifically for neoplastic tissues.

12. Composition according to claim 11 in which the screening molecule is selected from the sugar derivatives and in particular mannose.

13. Composition according to any one of claims 5 to 12 for use as a medicament.

14. Composition according to claim 13 for use for treating and/or preventing and/or detecting cancer.
